# EUROPEAN PATENT SPECIFICATION

(11) **EP 2 066 643 B1**
(45) Date of publication and mention of the grant of the patent: **16.03.2011**
(21) Application number: 07847257.8
(22) Date of filing: 21.11.2007
(51) Int. Cl.: C07D 237/08, C07D 403/12, C07D 409/12, C07D 237/06, A01N 43/58

(54) **PYRIDAZIN-4-YLMETHYL-SULFONAMIDES USED AS FUNGICIDES AND AGAINST ARTHROPODS**
ALS FUNGIZIDE UND GEGEN GLIEDERFÜSSER EINSETZBARE PYRIDAZIN-4-YL-METHYL-SULFONAMIDE
PYRIDAZINE-4-YLMETHYL-SULFONAMIDES UTILISÉS EN TANT QUE FONGICIDES ET COMPOSÉS ANTI-ARTHROPODES

(30) Priority: 22.11.2006 EP 06124590
(43) Date of publication of application: 10.06.2009
(73) Proprietor: BASF SE, 67056 Ludwigshafen (DE)
(72) Inventor: LOHMANN, Jan Klaas, 68159 Mannheim (DE); GRAMMENOS, Wassilios, 67071 Ludwigshafen (DE); PUHL, Michael, 68623 Lampertheim (DE); DIETZ, Jochen, 76227 Karlsruhe (DE); MÜLLER, Bernd, 67227 Frankenthal (DE); RHEINHEIMER, Joachim, 67063 Ludwigshafen (DE); RENNER, Jens, 67098 Bad Dürkheim (DE); VRETTOU, Marianna, 68165 Mannheim (DE); ULMSCHNEIDER, Sarah, 67098 Bad Dürkheim (DE); GROTE, Thomas, 67157 Wachenheim (DE)
(86) International application number: PCT/EP2007/062631
(87) International publication number: WO 2008/062012

(56) References cited:
- WO-A-2005/033081
- WO-A1-2006/097488
- WO-A1-2006/097489
- COWDEN, CAMERON J.: "Use of N-Protected Amino Acids in the Minisci Radical Alkylation" ORGANIC LETTERS , 5(23), 4497-4499 CODEN: ORLEF7; ISSN: 1523-7060, 2003, XP002424440 cited in the application

## Description

The present invention relates to novel pyridazin-4-ylmethyl-sulfonamide compounds, the N-oxides, and salts thereof and their use for combating arthropod pests and/or phytopathogenic harmful fungi, and also to compositions comprising, as active component, at least one such compound. The present invention also relates to a method for controlling arthropod pests.

Org. Lett., 5 (23), 4497 -4499, 2003 describes a process for preparing pyridazin-4-ylmethylamides of carboxylic acids and of p-toluenesulfonic acid by treating an N-protected amino acid with a pyridazine compound in the presence of ammonium peroxydisulfate and silver nitrate. The compound N-(3,6-dichloro-pyridazin-4ylmethyl)-4-methylbenzenesulfonamide is mentioned, inter alia.

WO 2005/33081 describes 4-pyridylmethyl sulfonamide compounds which are active against plant pathogenic fungi. With respect to their fungicidal activity, some of said 4-pyridylmethyl sulfonylamide are unsatisfactory, or they have unwanted properties such as low crop plant compatibility

WO 2006/097489 (PCT/EP/2006/060753) describes various 4-pyridylmethylamides of biphenylsulphonic acid, wherein the biphenyl moiety may carry substituents at the phenyl ring of the biphenyl moiety at the sulfonamide group. The compounds are used for combating arthropod pests and for protecting materials against infestation and/or destruction by said pests.

WO 2006/097488 (PCT/EP/2006/060752) inter alia describes arthropod quinolone compounds of the formula (A), 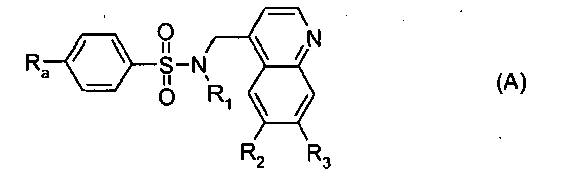 wherein R₂ and R₃ are both hydrogen, halogen, methoxy or trifluoromethoxy, R₁ is hydrogen or methyl and Rₐ is selected from phenyl which may be unsubstituted or may carry one substituent selected from chloro, C₁-C₄-alkyl, methoxy, trifluoromethoxy or phenyl.

WO 2007/104726 (corresponds to US 60/782429) describes specific quinoline methylsulfonamides carrying a biphenyl moiety at the sulfonamide group wherein the phenylene moiety of biphenyl is unsubstituted.

PCT/EP2007/058348 (corresponds to EP 06119331.4) describes thiophene-sulfonic acid picolyl amide compounds and their use for combating arthropod pests. Said compounds have also a fungicidal activity.

Based on this, there is ongoing need to provide compounds which are useful for combating harmful arthropodes such as insects and arachnids and/or harmful fungi.

This object is, surprisingly, achieved by pyridazin-4-ylmethyl-sulfonamide compounds of the general formula (I) described below 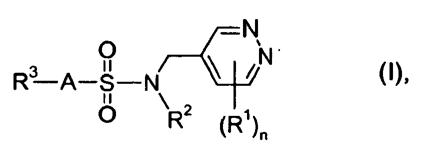 where:
- n: is zero, one, two or three;
- R¹: is halogen, C₁-C₄-alkyl, C₁-C₄-haloalkyl, C₁-C₄-alkoxy, C₁-C₄-haloalkoxy, C₁-C₄- alkylthio, C₁-C₄-haloalkylthio, C₁-C₄-alkylsulfinyl, C₁-C₄-haloalkylsulfinyl, C₁-C₄- alkylsulfonyl, C₁-C₄-haloalkylsulfonyl, amino, C₁-C₄-alkylamino, di(C₁-C₄- alkyl)amino, C₁-C₄-alkoxy-C₁-C₄-alkyl, C₁-C₄-haloalkoxy-C₁-C₄-alkyl, C₂-C₄- alkenyl, C₂-C₄-alkynyl, C₃-C₈-cycloalkyl or C₁-C₄-alkyl-C₃-C₈-cycloalkyl; and/or one-radical R¹ that is bound to a carbon atom adjacent to a nitrogen atom of the pyridazine ring may form together with said carbon atom and nitrogen atom a fused five-membered aromatic heterocycle, which may contain one, two or three further nitrogen atoms as ring members; or two radicals R¹ that are bound to adjacent carbon atoms of the pyridazine ring may form together with said carbon atoms a fused benzene ring, a fused saturated or partially unsaturated 5-, 6-, or 7-membered carbocycle or a fused 5-, 6-, or 7-membered heterocycle containing one, two or three heteroatoms selected from the group consisting of 2 nitrogen, 1 oxygen and 1 sulfur atoms as
- R²: ring members, it being possible for the fused ring to carry one or two radicals selected from the group consisting of halogen, C₁-C₄-alkyl, halomethyl, C₁-C₄- alkoxy or halomethoxy; is hydrogen, C₁-C₄-alkyl, C₁-C₄-haloalkyl, C₁-C₄-alkoxy, C₁-C₄-haloalkoxy, C₁-C₄- alkylamino, di(C₁-C₄-alkyl)amino, C₁-C₄-alkoxy-C₁-C₄-alkyl, C₁-C₄-haloalkoxy-C₁- C₄-alkyl, C₂-C₄-alkenyl, C₂-C₄-haloalkenyl, C₂-C₄-alkynyl, C₃-C₈-cycloalkyl, C₁-C₄- alkyl-C₃-C₈-cycloalkyl or benzyl wherein the phenyl moiety of benzyl is unsubstituted or carries 1, 2 , 3, 4, or 5 substituents selected from the group consisting of cyano, halogen,-C₁-C₄-alkyl, C₁-C₄-haloalkyl, C₁-C₄-alkoxy, C₁-C₄- haloalkoxy, (C₁-C₄-alkoxy)carbonyl and di(C₁-C₄-alkyl)aminocarbonyl;
- A: is phenylene, 5- or 6-membered heteroarenediyl, where heteroarenediyl contains one, two, three or four heteroatoms selected from the group consisting of 1, 2, 3 or 4 nitrogen atoms, 1 oxygen atom and 1- sulfur atom, as ring members, and where phenylene and heteroarenediyl for their part are unsubstituted or carry 1, 2, 3 or 4 substituents R^{A}, each selected from the group consisting of cyano, halogen, C₁-C₄-alkyl, C₁-C₄-haloalkyl, C₁-C₄-alkoxy, C₁-C₄-haloalkoxy, C₂-C₄- alkenyl, C₂-C₄-haloalkenyl, C₂-C₄-alkynyl, C₂-C₄-haloalkynyl, (C₁-C₄- alkoxy)carbonyl and di(C₁-C₄-alkyl)aminocarbonyl, or '
- A: is C₁-C₈-alkanediyl, C₁-C₈-haloalkanediyl, C₂-C₈-alkenediyl, C₂-C₈-haloalkenediyl, C₂-C₈-alkynediyl, or C₂-C₈-haloalkynediyl, wherein the six last-mentioned radicals are unsubstituted or carry one, two or three substituents R^{AA}, each selected from the group consisting of cyano, C₁-C₄-alkoxy, C₁-C₄-haloalkoxy, (C₁-C₄- alkoxy)carbonyl and di(C₁-C₄-alkyl)aminocarbonyl;
- R³: is hydrogen, C₁-C₄-alkoxy, C₁-C₄-haloalkoxy, C₁-C₄-alkylthio, C₁-C₄-haloalkylthio, C₁-C₄-alkylamino, di(C₁-C₄-alkyl)amino, (C₁-C₄-alkyl)carbonyl, (C₁-C₄- alkoxy)iminomethyl, acryloyl (vinylcarbonyl), C₃-C₈-cycloalkyl, C₁-C₄-alkyl-C₃-C₈- cycloalkyl, or C₅-C₈-cycloalkenyl; phenyl, benzyl, phenoxy, phenylthio, a 5- or 6-membered heteroaryl radical, wherein the heteroaryl ring has 1, 2, 3 or 4 heteroatoms selected from the group consisting of 1, 2, 3 or 4 nitrogen atoms, 1 oxygen atom and 1 sulfur atom, as ring members, it being possible for the heteroaryl ring and the phenyl ring of phenyl, benzyl, phenoxy and phenylthio to be unsubstituted or substituted by one, two or three substituents R^{B}, each selected from the group consisting of cyano, nitro, amino, halogen, C₁-C₄-alkyl, C₁-C₄-haloalkyl, C₁-C₄-alkoxy, C₁-C₄- haloalkoxy, (C₁-C₄-alkoxy)carbonyl, di(C₁-C₄-alkyl)aminocarbonyl, C₁-C₄-alkylthio, C₁-C₄-alkylsulfinyl and C₁-C₄-alkylsulfonyl; or a 5- or 6-membered heteroaryloxy radical or a 5- or 6-membered heteroarylthio radical, wherein the heteroaryl ring in the two aforementioned radicals has 1, 2, 3 or 4 heteroatoms selected from the group consisting of 1, 2, 3, or 4 nitrogen atoms, 1 oxygen atom and 1 sulfur atom, as ring members, it being possible for said heteroaromatic ring to carry one, two or three substituents R^{B}; or if A is phenylene or 5- or 6-membered heteroarenediyl, the radical R³ together with a radical R^{A} may form together with the carbon atoms to which they are bound a fused benzene ring, wherein the fused benzene ring may be unsubstituted or may carry 1, 2 or 3 substituents selected, independently from one-another, from the group consisting of halogen, cyano, nitro, C₁-C₄-alkyl, C₁- C₄-haloalkyl, C₁-C₄-alkoxy, C₁-C₄-haloalkoxy, (C₁-C₄-alkoxy)carbonyl and di(C₁- C₄-alkyl)aminocarbonyl, or if A is phenylene or 5- or 6-membered heteroarenediyl, the radical R³ can also be C₁-C₄-alkyl, C₂-C₄-alkenyl or C₂-C₄-alkynyl;
the N-oxides, and the salts of the compounds of the formula (I), except for the compound N-(3,6-dichloro-pyridazin-4ylmethyl)-4-methylbenzenesulfonamide.

Accordingly, the present invention relates to pyridazin-4-ylmethyl-sulfonamide compounds of the general formula (I) and the N-oxides and salts thereof. Moreover, the invention relates to an agricultural composition, which comprises a solid or liquid carrier and at least one compound of the formula (I) or an N-oxide or an agriculturally acceptable salt thereof. Furthermore, the present invention relates to a process for preparing compounds of the formula (I).

The compounds of the present invention are useful for combating phytopathogenic harmful fungi. Therefore the present invention also relates to the use of pyridazin-4-ylmethyl-sulfonamide compounds of the general formula (I), their N-oxides and salts for combating phytopathogenic harmful fungi.

The present invention furthermore relates to a method for the treatment of phytopathogenic harmful fungi, which process comprises treating the fungi or the materials, plants, the soil or seeds to be protected against fungal attack, with an effective amount of at least one compound of the formula (I) or an or an N-oxide or an agriculturally acceptable salt thereof.

The compounds of the present invention are also useful for combating arthropod pests, especially insects and arachnids. Therefore the present invention also relates to the use of compounds of the general formula (I) and their N-oxides and salts for protecting seed, the seedlings' roots and shoots from infestation by phytopathogenic harmful fungi and/or arthropod pests. The present invention furthermore relates to a method for combating arthropod pests, which comprises contacting said pests, their habitat, breeding ground, food supply, plant, seed, soil, area, material or environment in which the arthropod pests are growing or may grow, or the materials, plants, seeds, soils, surfaces or spaces to be protected from an attack of or infestation by said pests, with a pesticidally effective amount of at least one compound of the formula (I), or a N-oxide or an agriculturally acceptable salt thereof or with a composition comprising at least one compound of the formula (I), and/or a N-oxide or a salt thereof. Furthermore, the present invention relates to a method of protecting growing plants from attack or infestation by arthropod pests, which comprising contacting a crop with a pesticidally effective amount of at least one compound of the formula (I) or a N-oxide or an agriculturally acceptable salt thereof.

Furthermore, the present invention relates to a method for protecting seeds from infestation by arthropod pests and of the seedlings' roots and shoots from infestation by arthropod pests, which comprises contacting the seed or of the seedlings' roots and shoots with a pesticidally effective amount of at least one compound of the formula (I), or an N-oxide or an agriculturally acceptable salt thereof.

The present invention also relates to a method for protecting non-living materials from attack or infestation by arthropod pests, which comprises contacting the non-living material with a pesticidally effective amount of at least one compound of the formula (I), or an N-oxide or an agriculturally acceptable salt thereof.

The present invention also relates to seeds comprising a compound of the formula (I), or an N-oxide or an agriculturally acceptable salt thereof, in an amount of from 0.1 g to 10 kg per 100 kg of seed.

Depending on the substitution pattern, the compounds of the formula (I) and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

The compounds of the formula (I) can be present in different crystal modifications whose biological activity may differ. They also form part of the subject matter of the present invention.

Agriculturally useful salts of the compounds (I) encompass especially the salts of those cations or the acid addition salts of those acids whose cations and anions, respectively, have no adverse effect on the pesticidal action of the compounds (I). Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting a compound of formula (I) with an.acid of the corresponding anion, preferably of hydrochloric acid, hydrobromic acid, sulfuric acid, phosphoric acid or nitric acid.

Veterinarily acceptable salts of the compounds of formula (I) encompass especially the salts of those cations or the acid addition salts which are known and accepted in the art for the formation of salts for veterinary use. Suitable acid addition salts, e.g. formed by compounds of formula (I) containing a basic nitrogen atom, e.g. an amino group, include salts with inorganic acids, for example hydrochlorids, sulphates, phosphates, and nitrates and salts of organic acids for example acetic acid, maleic acid, dimaleic acid, fumaric acid, difumaric acid, methane sulfenic acid, methane sulfonic acid, and succinic acid.

In the definitions of the variables given above, collective terms are used which are generally representative for the substituents in question. The term Cₙ-Cₘ indicates the number of carbon atoms possible in each case in the substituent or substituent moiety in question.

The term "halogen" refers to fluorine, chlorine, bromine and iodine.

The term "C₁-C₄-alkyl" refers to a straight-chained or branched saturated hydrocarbon group having 1 to 4 carbon atoms, for example methyl, ethyl, propyl, 1-methylethyl, butyl, 1-methylpropyl, 2-methylpropyl, and 1,1-dimethylethyl.

The term "C₁-C₄-haloalkyl" refers to a straight-chained or branched alkyl group having 1 to 4 carbon atoms (as defined above), wherein some or all of the hydrogen atoms in these groups may be replaced by halogen atoms as mentioned above, for example chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, CH₂-C₂F₅, CF₂-C₂F₅, CF(CF₃)₂, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl, and the like.

The term "C₁-C₄-alkoxy" refers to a straight-chain or branched alkyl group having 1 to 4 carbon atoms (as defined above) which is bonded via an oxygen, at any position in the alkyl group, for example methoxy, ethoxy, n-propoxy, 1-methylethoxy, butoxy, 1-methylpropoxy, 2-methylpropoxy or 1,1-dimethylethoxy.

The term "C₁-C₄-haloalkoxy" refers to a C₁-C₄-alkoxy radical as defined above which is partly or fully substituted by fluorine, chlorine, bromine and/or iodine, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro-ethoxy, OC₂F₅, 2-fluoropropoxy, 3-fluoropropoxy, 2,2-difluoropropoxy, 2,3-difluoro-propoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF-C₂F₅, 1-(CH₂F)-2-fluoroethoxy, 1-(CH₂Cl)-2-chloroethoxy, 1-(CH₂Br)-2-bromoethoxy, 4-fluorobutoxy, 4-chlorobutoxy, 4-bromobutoxy or nonafluorobutoxy.

The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above).

Accordingly, the term "C₁-C₄-alkylthio" as used herein refers to straight-chain or branched alkyl groups having 1 to 4 carbon atoms (as defined above) bonded through a sulfur atom, at any position in the alkyl group, for example methylthio, ethylthio, propylthio, isopropylthio, and n-butylthio.

The term "C₁-C₄-alkylsulfinyl" refers to straight-chain or branched alkyl group having 1 to 4 carbon atoms (as defined above) bonded through a -S(=O) moiety, at any position in the alkyl group, for example methylsulfinyl and the like.

The term "C₁-C₄-haloalkylsulfinyl" refers to straight-chain or branched haloalkyl group having 1 to 4 carbon atoms (as defined above) bonded through a -S(=O) moiety, at any position in the alkyl group.

The term "C₁-C₄-alkylsulfonyl" refers to straight-chain or branched alkyl group having 1 to 4 carbon atoms (as defined above) bonded through a -S(=O)₂ moiety, at any position in the alkyl group, for example methylsulfonyl.

The term "C₁-C₄-haloalkylsulfonyl" refers to straight-chain or branched haloalkyl group having 1 to 4 carbon atoms (as defined above) bonded through a -S(=O)₂ moiety, at any position in the alkyl group.

The term "alkylamino" refers to an amino radical carrying one C₁-C₄-alkyl group (as defined above) as substituent, for example methylamino, ethylamino, propylamino, 1-methylethylamino, butylamino, 1-methylpropylamino, 2-methylpropylamino, 1,1-dimethylethylamino and the like.

The term "di(C₁-C₄-alkyl)amino" refers to an amino radical carrying two identical or different C₁-C₄-alkyl groups (as defined above) as substituents, for example dimethylamino, diethylamino, di-n-propylamino, diisopropylamino, N-ethyl-N-methylamino, N-(n-propyl)-N-methylamino, N-(isopropyl)-N-methylamino, N-(n-butyl)-N-methylamino, N-(n-pentyl)-N-methylamino, N-(2-butyl)-N-methylamino, N-(isobutyl)-N-methylamino, and the like.

The term "C₁-C₄-alkoxycarbonyl" refers to a C₁-C₄-alkoxy radical as defined above which is attached via a carbonyl group.

The term "di(C₁-C₄-alkyl)aminocarbonyl" refers to a di(C₁-C₄)alkylamino radical as defined above which is attached via a carbonyl group.

The terms "phenoxy" and "phenylthio",' respectively, refer to a phenyl radical which is attached via an oxygen and sulfur atom, respectively.

The term "C₂-C₄-alkenyl" refers to a branched or unbranched unsaturated hydrocarbon radical having 2 to 4 carbon atoms and a double bond in any position, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl.

The term "C₂-C₄-haloalkenyl" refers to an unsaturated straight-chain or branched hydrocarbon radical having from 2 to 4 carbon atoms and one double bond in any position (as defined above), where some or all of the hydrogen atoms in these groups may be replaced by halogen atoms as specified above, in particular fluorine, chlorine and bromine.

The term "C₂-C₄-alkynyl" refers to a branched or unbranched unsaturated hydrocarbon radical having 2 to 4 carbon atoms and containing at least one triple bond, such as ethynyl, 1-propynyl, 2-propynyl (propargyl), 1-butynyl, 2-butynyl, 3-butynyl, 1-methyl-2-propynyl.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-cycloalkenyl" refers to monocyclic monounsaturated hydrocarbon radicals having from 3 to 8, preferably from 5 to 6, carbon ring members, such as cyclopenten-1-yl, cyclopenten-3-yl, cyclohexen-1-yl, cyclohexen-3-yl, cyclohexen-4-yl and the like

The term "C₁-C₄-alkyl-C₃-C₈-cycloalkyl" refers to a cycloalkyl radical having 3 to 8 carbon atoms (as defined above), wherein one hydrogen atom of the cycloalkyl radical is replaced by a C₁-C₄-alkyl group (as defined above).

The term "five- or six-memberd heterocycle" which contains one, two, three or four heteroatoms from the group consisting of O, N and S, is to be understood as meaning both saturated, partially unsaturated and aromatic heterocycles having 5 or 6 ring atoms, including:
- 5- or 6-membered heterocyclyl which contains one, two or three nitrogen atoms and/or one oxygen or sulfur atom or one or two oxygen and/or sulfur atoms, and which is saturated or partially unsaturated, for example 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 2-pyrrolin-2-yl, 2-pyrrolin-3-yl, 3-pyrrolin-2-yl, 3-pyrrolin-3-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl and 2-piperazinyl;
- 5-membered aromatic heterocyclyl (heteroaryl) which contains one, two, three or four nitrogen atoms or one, two or three nitrogen atoms and one sulfur or oxygen atom: 5-membered heteroaryl groups which, in addition to carbon atoms, may contain one to four nitrogen atoms or one to three nitrogen atoms and one sulfur or oxygen atom as ring members, for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which contains one, two, three or four nitrogen atoms: 6-membered heteroaryl groups which, in addition to carbon atoms, may contain one, two, three or four nitrogen atoms as ring members, for example 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

Fused 5- or 6-membered carbocycle means a hydrocarbon ring which shares two adjacent carbon atoms with another ring, examples being cylopentane, cyclopentene, cyclohexane, cyclohexene and benzene. Examples for 5- or 6-membered heterocycles which contain a contain a fused 5 or 6 membered carbocyclic ring as mentioned above are indolyl, indolinyl, isoindolinyl, benzpyrazolyl, benzimidazolyl, benzotriazolyl, quinolinyl, 1,2,3,4-tetrahydroquinolinyl, isoquinolinyl, phthalazinyl, quinazinyl, quinazolinyl, cinnolinyl, benzofuranyl, benzo-thiophenyl, benzopyranyl, dihydrobenzopyranyl, benzothiopyranyl, 1,3-benzodioxolyl, benzoxazolyl, benzthiazolyl, benzisoxazolyl and 1,4-benzodioxanyl.

The term "5-, 6- or 7-membered carbocycle" comprises monocyclic nonaromatic saturated or partially unsaturated carbocyclic rings having 5, 6 or 7 ring members. Examples for non-aromatic rings include cyclopentyl, cyclopentenyl, cyclopentadienyl, cyclohexyl, cyclohexenyl, cyclohexadienyl, cycloheptyl, cycloheptenyl, cycloheptadienyl and the like.

The term "C₁-C₈-alkanediyl" refers to a divalent, branched, or straight-chain saturated hydrocarbon radical having 1 to 8 carbon atoms, derived by the removal of one hydrogen atom from each of two different carbon atoms of a parent C₁-C₈-alkane, or by the removal of two hydrogen atoms from a single carbon atom of a parent C₁-C₈-alkane, for example, methanediyl, ethan-1,1-diyl, ethan-1,2-diyl, propan-1,1-diyl, propan-1,2-diyl, propan-2,2-diyl, propan-1,3-diyl, butan-1,1-diyl, butan-1,2-diyl, butan-1,3-diyl, butan-1,4-diyl, butan-2,2-diyl, 2-methyl-propan-1,1-diyl, 2-methyl-propan-1,2-diyl, and the like.

The term "C₁-C₈-haloalkanediyl" refers to a divalent, branched, or straight-chain saturated hydrocarbon group having 1 to 8 carbon atoms, as defined above, wherein some or all of the hydrogen atoms in these groups may be replaced by halogen atoms as mentioned above.

The term "C₂-C₈-alkenediyl" refers to a divalent, branched, or straight-chain unsaturated hydrocarbon group having 2 to 8 carbon atoms, derived by the removal of one hydrogen atom from each of two different carbon atoms of a parent C₂-C₈-alkene, or by the removal of two hydrogen atoms from a single carbon atom of a parent C₂-C₈-alkene, for example, ethen-1,2-diyl, ethen-1,1-diyl, prop-1-en-1,1-diyl, prop-2-en-1,2-diyl, prop-1-en-1,3-diyl, propen-3,3-diyl, propen-2,2-diyl, but-2-en-1,4-diyl and the like.

The term "C₂ -C₈-haloalkenediyl" refers to a divalent, branched, or straight-chain unsaturated hydrocarbon group having 2 to 8 carbon atoms, as defined above, wherein some or all of the hydrogen atoms in these groups may be replaced by halogen atoms as mentioned above.

The term "C₂-C₈-alkynediyl" refers to a divalent, branched, or straight-chain unsaturated hydrocarbon radical having 2 to 8 carbon atoms, derived by the removal of one hydrogen atom from each of two different carbon atoms of a parent C₂-C₈-alkyne, or by the removal of two hydrogen atoms from a single carbon atom of a parent C₂-C₈-alkyne, for example, prop-2-yn-1,1-diyl, prop-2-yn-1,3-diyl, prop-1-yn-1,3-diyl, but-1-yn-1,3-diyl, but-1-yn-1,4-diyl, but-2-yn-1,4-diyl and the like.

The term "C₂-C₈-haloalkynediyl" refers to a divalent, branched, or straight-chain unsaturated hydrocarbon radical having 2 to 8 carbon, as defined above, wherein some or all of the hydrogen atoms in these groups may be replaced by halogen atoms as mentioned above.

The term "phenylene" refers to 1,2-phenylene (o-phenylene), 1,3-phenylene (m-phenylene) and 1,4-phenylene (p-phenylene).

The term "5- or 6-membered heteroarenediyl" refers to a divalent radical derived from aromatic heterocycles having 5 or 6 ring atoms, as defined above, aromatic heterocycles having two points of attachment. Examples of heteroarenediyl radicals are, for example, divalent radicals derived from pyridine, pyrimidine, pyridazine, 1,2,3-triazine, 1,2,4-triazine, 1,2,3,4-tetrazine, furan, thiophene, pyrrole, thiazole, thiadiazole, pyrazole, imidazole, triazole, tetrazole, oxazole, isoxazole, isothiazole, oxadiazole and the like. The aforementioned groups can be C-attached or N-attached where such is possible. For example, a group derived from pyrrole, imidiazole or pyrazole can be N-attached or C-attached.

The term "if A is phenylene or 5- or 6-membered heteroarenediyl, the radical R³ together with a radical R^{A} may form together with the carbon atoms to which they are bound a fused benzene ring" refers to a bicyclic ring system, wherein phenylene and heteroarenediyl, respectively, carry a fused-on benzene ring. Examples of fused bicyclic rings include naphthalene, benzo[b]furan, benzo[b]thiophene, benzimidazole, benzoxazole, benzthiazole, indole, quinoline and the like. The fused-on benzene ring can be unsubstituted or substituted by 1, 2, 3 radicals substituents selected, independently from one another, from the group consisting of halogen, cyano, nitro, C₁-C₄-alkyl, C₁-C₄-haloalkyl, C₁-C₄-alkoxy, C₁-C₄-haloalkoxy, (C₁-C₄-alkoxy)carbonyl and di(C_{I}-C₄-alkyl)aminocarbonyl. In addition, the phenylene moiety and the heteroarenediyl moiety of the bicyclic ring systems can be unsubstituted or substituted with 1, 2 or 3 substituents R^{A}.

The term "one radical R¹ that is bound to a carbon atom adjacent to a nitrogen atom of the pyridazine ring may form together with said carbon atom and nitrogen atom a fused five-membered aromatic heterocycle, which may contain one, two or three further nitrogen atoms as ring members" refers to a radical of the formula (B) 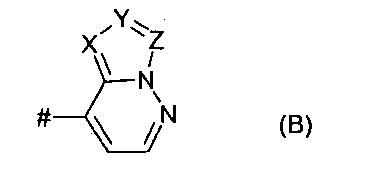 in which # denotes the point of attachment to the skeleton of the remaining molecule, X, Y and Z are, independently of one another, selected from CH and nitrogen.

As regards the pesticidal activity of the compounds of the formula (I), preference is given to those compounds of the formula (I), wherein the variables n, R¹, R², R³, and A have independently of each other or more preferably in combination the following meanings.

Preference is given to compounds of the formula (I), in which A is phenylene or heteroarenediyl, as defined above, which both may be unsubstituted or may carry one, two or three substituents R^{A}.

Particular preference is given to compounds of the formula (I), in which A is phenylene, which is unsubstituted or substituted by one, two or three substituents R^{A}, with 1,3-phenylene or 1,4-phenylene being more preferred. Most preference is given to compounds of the formula (I), in which A is 1,4-phenylene, which may be unsubstituted or substituted by one, two or three substituents R^{A}, especially preferably A is 1,4-phenylene, which is unsubstituted.

Likewise, particular preference is given to compounds of the formula (I), in which A is heteroarenediyl selected from the group consisting of pyridinediyl, pyrimidinediyl, pyridazinediyl, pyrazinediyl, pyrrolediyl, pyrazolediyl, imidazolediyl, triazolediyl, tetrazolediyl, triazine, furandiyl, thiophenediyl, thiazolediyl, oxazolediyl, isoxazolediyl, isothiazolediyl, thiadiazolediyl, and oxadiazole, and where the 18 last-mentioned radicals are unsubstituted or carry one, two or three substituents R^{A}. If one point of attachement is located on a nitrogen atom of the heteroarenediyl radical, said nitrogen atom is attached either to the sulfur atom of the sulfonamide group or to R³, with the point of attachment to R³ being more preferred. Examples of heteroarenediyl radicals A are pyridin-2,3-diyl, pyridin-2,4-diyl, pyridin-2,5-diyl, pyridin-2,6-diyl, pyridin-3,5-diyl, pyrimidin-2,4-diyl, pyrimidin-2,5-diyl, pyrimidin-4,6-diyl, pyridazin-3,5-diyl, pyridazin-3,6-diyl, pyrazin-2,6-diyl, pyrazin-2,5-diyl, 1,2,3-triazin-4,5-diyl, 1,2,3-triazin-4,6-diyl 1,2,4-triazin-3,6-diyl, 1,2,4-triazin-3,5-diyl 1,3,5-triazin-2,4-diyl, furan-2,5-diyl, furan-2,4-diyl, furan-3,5-diyl, thiophen-2,5-diyl, thiophen-2,4-diyl, thiophen-3,5-diyl, pyrrol-2,4-diyl, pyrrol-2,5-diyl, [1H]-pyrrol-1,3-diyl, thiazol-2,4-diyl, thiazol-2,5-diyl, pyrazol-3,4-diyl, pyrazol-3,5-diyl, [1H]-pyrazol-1,3-diyl, [1H]-pyrazol-1,4-diyl, imidazol-2,4-diyl, imidazol-2,5-diyl, [1H]-imidazol-1,4-diyl, [1H]-1,2,3-triazol-4,5-diyl, [1H]-1,2,3-triazol-1,5-diyl, [1H]-1,2,3-triazol-1,4-diyl, [1H]-1,2,4-triazol-3,5-diyl, [1H]-1,2,4-triazol-1,3-diyl, [1H]-1,2,3-triazol-1,5-diyl, [1H]-1,2,3,4-tetrazol-4,5-diyl, 2,4-oxazoldiyl, 2,5-oxazoldiyl, 4,5-oxazoldiyl, isoxazol-3,4-diyl, isoxazol-3,5-diyl, isoxazol-4,5-diyl, isothiazol-3,4-diyl, isothiazol-3,5-diyl, isothiazol-4,5-diyl, 1,2,3-oxadiazol-4,5-diyl, 1,2,4-oxadiazol-3,5-diyl, 1,3,4-oxadiazol-2,5-diyl, 1,2,5-oxadiazol-3,4-diyl, 1,2,3-thiadiazol-4,5-diyl, 1,2,4-thiadiazol-3,5-diyl, 1,3,4-thiadiazol-2,5-diyl and 1,2,5-thiadiazol-3,4-diyl, and each of which is optionally substituted by one, two or three substituents R^{A}.

Amongst compounds of the formula (I), in which A is heteroarenediyl, particular preference is given to those, in which A is thiophenediyl, thiazolediyl, oxazolediyl, pyrazolediyl or pyridinediyl, where each of the aforementioned five radicals are unsubstituted or carry one, two or three substituents R^{A}.

Amongst compounds of the formula (I), in which A is heteroarenediyl, most preference is given to those, in which A is selected from the group consisting of thiophene-2,5-diyl, thiophene-2,4-diyl, thiophene-3,5-diyl, thiazole-2,5-diyl, thiazole-2,4-diyl, oxazole-2,5-diyl, oxazole-2,4-diyl, pyrazole-3,5-diyl, pyrazole-1,3-diyl, pyrazole-1,4-diyl, pyridine-2,5-diyl, pyridine-2,6-diyl, pyridine-2,4-diyl, pyridine-3,5-diyl, wherein heteroarenediyl is unsubstituted or carries one, two or three substituents R^{A}.

Particularly preferred embodiments of the invention relate to compounds of the formula (I), in which A is one of the radicals A-1 to A-30 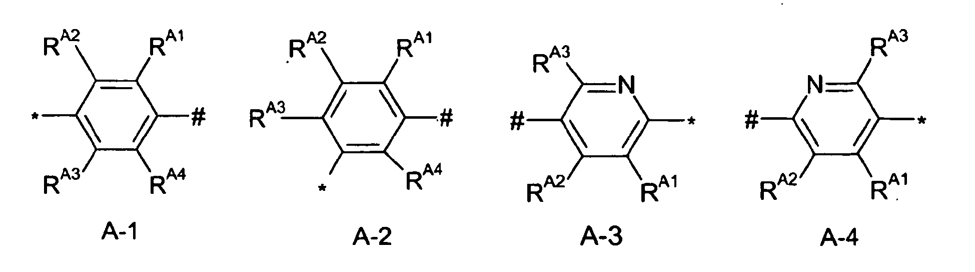 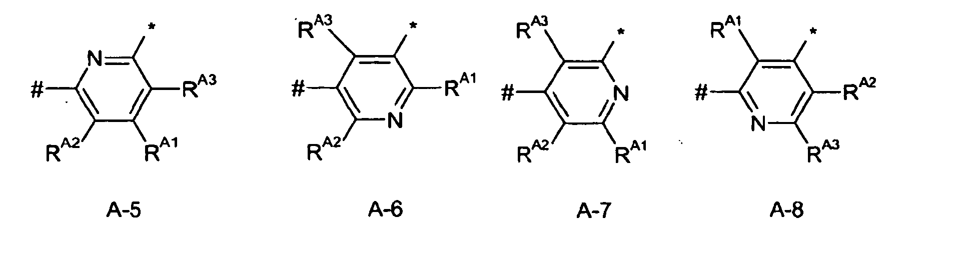 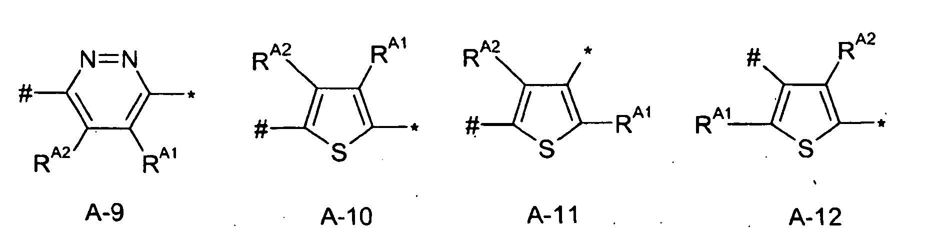 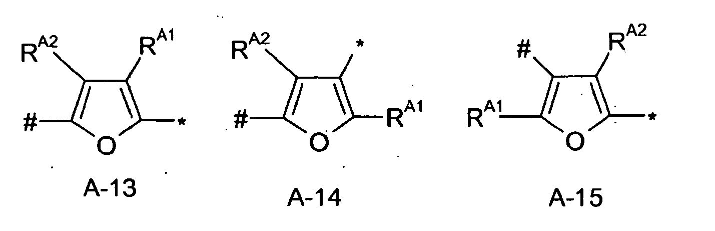 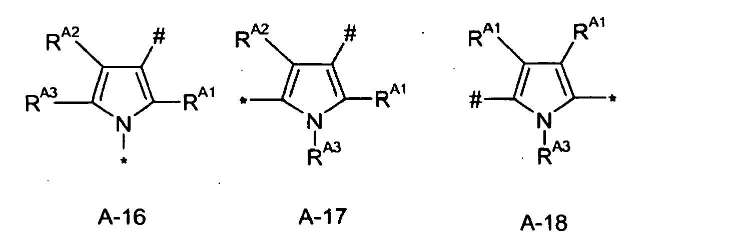 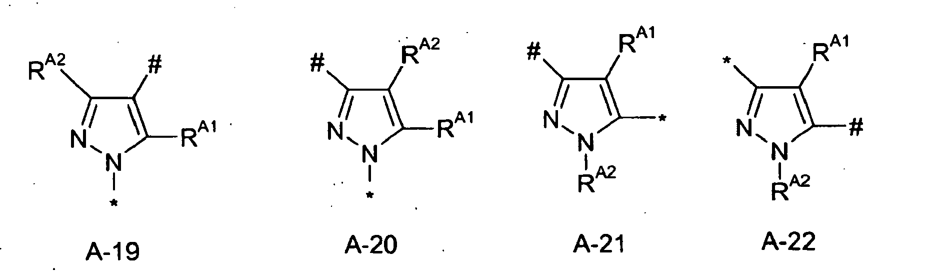 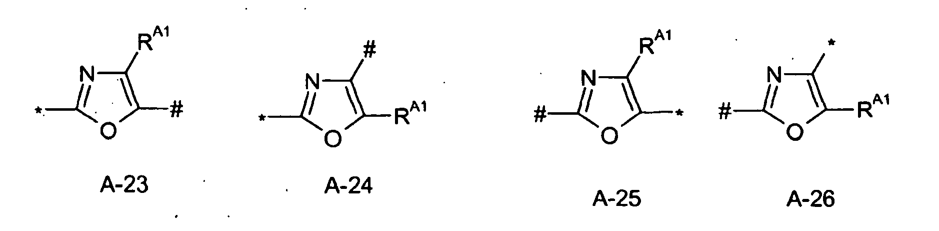 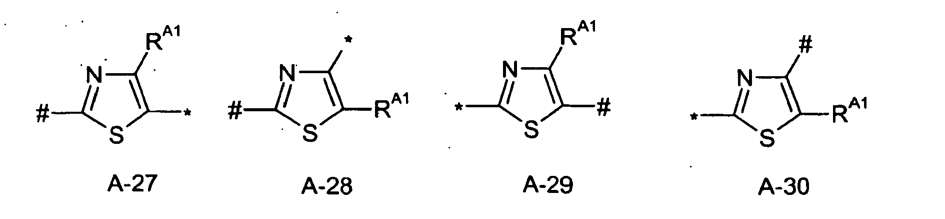 in which
# indicates the point of attachment to the sulfur atom of the sufonamide group;
* indicates the point of attachment to R³; and
R^{A1},R^{A2}, R^{A3} and R^{A4} are each independently hydrogen or have one of the definitions specified for R^{A}, especially those being preferred.

If R^{A} is present, preferred substituents R^{A} are selected, independently of one another, from halogen, such as fluorine or chlorine, cyano, C₁-C₄-alkyl, such as methyl or ethyl, C₁-C₄-alkoxy, such as methoxy, C₁-C₄-haloalkoxy such as C₁-C₂-fluoroalkoxy, and C₁-C₄-haloalkyl such as C₁-C₂-fluoroalkyl.

A further preferred embodiment of the invention relates to compounds of the formula (I) in which A is C₂-C₄-alkenediyl, C₂-C₄-haloalkenediyl, C₂-C₄-alkynediyl or C₂-C₄-haloalkynediyl, and where the four last-mentioned radicals are unsubstituted or carry one, two, or three substituents R^{AA}. Preferred substituents R^{AA} are selected from C₁-C₄-alkoxy and C₁-C₄-haloalkoxy. R^{AA} is in particular C₁-C₂-alkoxy, such as methoxy, or C₁-C₂-fluoroalkoxy, such as trifluoromethoxy. Among these, particular preference is given to compounds of the formula (I), in which A is ethen-1,2-diyl or ethyn-1,2-diyl, which may be unsubstituted or carry 1 substituent R^{AA} as defined above.

Preferred are compounds of the formula (I), wherein R³ is phenyl or phenoxy, where the two last-mentioned radicals are unsubstituted or carry one, two, or three substituents R^{B}. Preferred substituents R^{B} are halogen, in particular fluorine or chlorine, C₁-C₂-alkyl such as methyl or ethyl, C₁-C₂-fluoroalkyl such as trifluoromethyl, C₁-C₂-alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁-C₂-alkoxy)carbonyl, in particular methoxycarbony, or di(C₁-C₂-alkyl)aminocarbonyl, such as dimethylaminocarbonyl. Especially preferred radicals R^{B} are each independently selected from fluorine and chlorine.

Very particular preference is given to compounds of the formula (I) in which R³ is phenyl, which is substituted by at least one substituent R^{B}. In this case, especially preferably, at least one of the radicals R^{B} is arranged in the para-position relative to the bonding site of phenyl to the A-moiety. In this embodiment, preferred substituents R^{B} are halogen, in particular fluorine or chlorine, C₁-C₂-alkyl such as methyl or ethyl, C₁-C₂-fluoroalkyl such as trifluoromethyl, C₁-C₂-alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁-C₂-alkoxy)carbonyl, in particular methoxycarbony, or di(C₁-C₂-alkyl)aminocarbonyl, such as dimethylaminocarbonyl. Especially preferred radicals R^{B} are each independently selected from fluorine and chlorine.

Likewise, most preferred are compounds of the formula (I), in which R³ is phenoxy, which is substituted by one substituent R^{B}, with chlorine and fluorine being most preferred substituents R^{B}. Likewise, most preferred are compounds of the formula (I) in which R³ is phenoxy, which is substituted with two, different or same substituents R^{B}. In this embodiment, preferred substituents R^{B} are halogen, in particular fluorine or chlorine, C₁-C₂-alkyl such as methyl or ethyl, C₁-C₂-fluoroalkyl such as trifluoromethyl, C₁-C₂-alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁-C₂-alkoxy)carbonyl, in particular methoxycarbony, or di(C₁-C₂-alkyl)aminocarbonyl, such as dimethylaminocarbonyl, with fluorine or chlorine being most preferred. In this embodiment, especially preferably at least one of the radicals R^{B} is located in the para-position relative to the bonding site of phenoxy to the A-moiety.

Preference is likewise given to compounds of the formula (I) in which R³-A are together C₂-C₈-alkenyl or C₂-C₈-alkynyl, where said alkenyl or said alkynyl are unsubstituted or substituted by R^{AA}, preferably by 1, 2, 3 or 4 substituents R^{AA}. Preferred substituents R^{AA} are selected from halogen, C₁-C₄-alkoxy and C₁-C₄-haloalkoxy. R^{AA} is in particular halogen, especially fluorine and chlorine, C₁-C₂-alkoxy, such as methoxy, or C₁-C₂-fluoroalkoxy, such as trifluoromethoxy. Among these, particular preference is given to compounds of the formula (I), in which R³-A are together C₂-C₄-alkenyl or C₂-C₄-alkynyl, which may be unsubstituted or substituted as defined above.

Particular preference is given to those compounds of the formula (I), in which the variables R³ and A have in combination the following meanings:
- R³: is phenyl, which is unsubstituted or substituted by at least one substituent R^{B}, preferably one or two. If R^{B} is present, especially preferably, one of the radicals R^{B} is arranged in the para-position relative to the bonding site of phenyl to the A- moiety. Preferred substituents R^{B} are halogen, in particular fluorine or chlorine, C₁-C₂-alkyl such as methyl or ethyl, C₁-C₂-fluoroalkyl such as trifluoromethyl, C₁- C₂₋alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁- C₂-alkoxy)carbonyl, in particular methoxycarbony, or di(C₁-C₂-
- A: alkyl)aminocarbonyl, such as dimethylaminocarbonyl. Especially preferred radicals R^{B} are each independently selected from fluorine and chlorine; and is phenylene, which is unsubstituted or substituted by one, two or three substituents R^{A}, in particular 1,3-phenylene or 1,4-phenylene, especially preferably 1,4-phenylene. With particular preference A is 1,4-phenylene, which is unsubstituted. If R^{A} is present, preferred substituents R^{A} are selected, independently of one another, from halogen, such as fluorine or chlorine, cyano, C₁-C₄-alkyl, such as methyl or ethyl, C₁-C₄-alkoxy, such as methoxy, C₁-C₄- haloalkoxy such as C₁-C₂-fluoroalkoxy, and C₁-C₄-haloalkyl such as C₁-C₂- fluoroalkyl.

Particular preference is likewise given to those compounds of the formula (I), in which the variables R³ and A have in combination the following meanings:
- R³: is phenyl, which is unsubstituted or substituted by at least one substituent R^{B}, preferably one or two. If R^{B} is present, especially preferably, one of the radicals R^{B} is arranged in the para-position relative to the bonding site of phenyl to the A- moiety. Preferred substituents R^{B} are halogen, in particular fluorine or chlorine, C₁-C₂-alkyl such as methyl or ethyl, C₁-C₂-fluoroalkyl such as trifluoromethyl, C₁- Cralkoxy such as methoxy, C₁-C₂-fluoroalkoxy, in particularC₁-Crfluoroalkoxy, such as trifluoromethoxy, C₁-C₂-alkoxycarbonyl such as methoxycarbonyl, (C₁- C₂-alkoxy)carbonyl, in particular methoxycarbony, or di(C₁-C₂- alkyl)aminocarbonyl, such as dimethylaminocarbonyl. Especially preferred radicals R^{B} are each independently selected from fluorine and chlorine; and
- A: is heteroarenediyl, which is unsubstituted or carries one, two or three substituents
R^{A}. If R^{A} is present, preferred substituents R^{A} are selected, independently of one another, from halogen, such as fluorine or chlorine, cyano, C₁-C₄-alkyl, such as methyl or ethyl, C₁-C₄-alkoxy, such as methoxy, C₁-C₄-haloalkoxy such as C₁-C₂-fluoroalkoxy, and C₁-C₄-haloalkyl such as C₁-C₂-fluoroalkyl. More preferably heteroarenediyl is selected from the group consisting of thiophene-2,5-diyl, thiophene-2,4-diyl, furan-2,5-diyl, furan-2,4-diyl, pyrrol-2,4-diyl; pyrrol-2,5-diyl, [1H]-pyrrol-1,3-diyl, thiazole-2,5-diyl, thiazole-2,4-diyl, oxazole-2,5-diyl, oxazole-2,4-diyl, pyrazole-3,5-diyl, pyrazole-1,3-diyl, pyrazole-1,4-diyl, pyridine-2,5-diyl, pyridine-2,6-diyl, pyridine-2,4-diyl, pyridine-3,5-diyl and pyridazine-3,6-diyl, wherein heteroarenediyl is unsubstituted or carries one, two or three substituents R^{A} , especially one of the radicals A-3, A-4, A-5, A-6, A-7, A-8, A-9, A-10, A-11, A-12, A-13, A-14, A-15, A-16, A-17, A-18, A-19, A-20, A-21, A-22, A-23, A-24, A-25, A-26, A-27, A-28, A-29 or A-30 as defined above.

Particular preference is likewise given to those compounds of the formula (I), in which the variables R³ and A have in combination the following meanings:
- R³: is phenoxy, which is unsubstituted; is phenoxy, which is substituted by one substituent R^{B}, with chlorine and fluorine being most preferred substituents R^{B}, or is phenoxy, which is substituted by two, different or same substituents R^{B}. In this embodiment, preferred substituents R^{B} are halogen, in particular fluorine or chlorine, C₁-C₂-alkyl such as methyl or ethyl, C₁-C₂-fluoroalkyl such as trifluoromethyl, C₁-C₂alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁-C₂-alkoxy)carbonyl, in particular methoxycarbony, or di(C₁- C₂-alkyl)aminocarbonyl, such as dimethylaminocarbonyl, with fluorine or chlorine being most preferred. If R^{B} is present, especially preferably one of the radicals R^{B} is arranged in the para-position relative to the bonding site of phenoxy to the A- moiety; and
- A: is phenylene, which is unsubstituted or substituted by one, two or three substituents R^{A}, in particular 1,3-phenylene or 1,4-phenylene, especially preferably 1,4-phenylene. With particular preference A is 1,4-phenylene, which is unsubstituted. If R^{A} is present, preferred substituents R^{A} are selected, independently of one another, from halogen, such as fluorine or chlorine, cyano, C₁-C₄-alkyl, such as methyl or ethyl, C₁-C₄-alkoxy, such as methoxy, C₁-C₄- haloalkoxy such as C₁-C₂-fluoroalkoxy, and C₁-C₄-haloalkyl such as C₁-C₂- fluoroalkyl.

Particular preference is likewise given to those compounds of the formula (I), in which the variables R³ and A have in combination the following meanings:
- R³: is phenoxy, which is unsubstituted, is phenoxy, which is substituted by one substituent R^{B}, with chlorine and fluorine being most preferred substituents R^{B}, or is phenoxy, which is substituted with two, different or same substituents R^{B}. In this embodiment, preferred substituents R^{B} are halogen, in particular fluorine or chlorine, C₁-C₂-alkyl such as methyl or ethyl, C₁-C₂-fluoroalkyl such as trifluoromethyl, C₁-C₂-alkoxy such as methoxy, C₁-C₂-fluoroalkoxy, in particular C₁-C₂-fluoroalkoxy, such as trifluoromethoxy, C₁-C₂-alkoxycarbonyl such as methoxycarbonyl, (C₁-C₂-alkoxy)carbonyl, in particular methoxycarbony, or di(C₁- C₂-alkyl)aminocarbonyl, such as dimethylaminocarbonyl, with fluorine or chlorine being most preferred. If R^{B} is present, especially preferably one of the radicals R^{B}

- A: is arranged in the para-position relative to the bonding site of phenoxy to the A- moiety; and is heteroarenediyl, which is unsubstituted or carries one, two or three substituents R^{A}. If R^{A} is present, preferred substituents R^{A} are selected, independently of one another, from halogen, such as fluorine or chlorine, cyano, C₁-C₄-alkyl, such as methyl or ethyl, C₁-C₄-alkoxy, such as methoxy, C₁-C₄-haloalkoxy such as C₁-C₂- fluoroalkoxy, and C₁-C₄-haloalkyl such as C₁-C₂-fluoroalkyl. More preferably heteroarenediyl is selected from the group consisting of thiophene-2,5-diyl, thiophene-2,4-diyl, furan-2,5-diyl, furan-2,4-diyl, pyrrol-2,4-diyl, pyrrol-2,5-diyl, [1H]-pyrrol-1,3-diyl, thiazole-2,5-diyl, thiazole-2,4-diyl, oxazole-2,5-diyl, oxazole- 2,4-diyl, pyrazole-3,5-diyl, pyrazole-1,3-diyl, pyrazole-1,4-diyl, pyridine-2,5-diyl, pyridine-2,6-diyl, pyridine-2,4-diyl, pyridine-3,5-diyl and pyridazine-3,6-diyl, wherein heteroarenediyl is unsubstituted or carries one, two or three substituents R^{A}, especially one of the radicals A-3, A-4, A-5, A-6, A-7, A-8, A-9, A-10, A-11, A- 12, A-13, A-14, A-15, A-16, A-17, A-18, A-19, A-20, A-21, A-22, A-23, A-24, A- 25, A-26, A-27, A-28, A-29 or A-30 as defined above.

A specific embodiment relates to compounds of the formula (I), in which A is a 5- or 6-membered heteroarenediyl, and the radical R³ together with a radical R^{A} form together with the carbon atoms to which they are bound a fused benzene ring, wherein the fused benzene ring may be unsubstituted or may carry 1, 2 or 3 substituents selected, independently from one another, from the group consisting of halogen, cyano, nitro, C₁-C₄-alkyl, C₁-C₄-haloalkyl, C₁-C₄-alkoxy, C₁-C₄-haloalkoxy, (C₁-C₄-alkoxy)carbonyl and di(C₁-C₄-alkyl)aminocarbonyl. In this case, preference is given to R³-A being together benzo[b]furan or benzo[b]thiophene, wherein the benzene moiety of benzo[b]furan and benzo[b]thiophene is unsubstituted or carries one or two substituents. Said substituents on the benzene moiety are preferable C₁-C₂-alkyl, especially methyl, or halogen, in particular chlorine. The furan moiety of benzo[b]furan and the thiophene moiety of benzo[b]thiophene can be unsubstituted or carry one substituent preferably selected from C₁-C₂-alkyl, especially methyl, or halogen, especially chlorine.

Preference is furthermore given to compounds of the formula (I) in which R² is hydrogen, C₁-C₄-alkyl, especially methyl, ethyl, n-propyl, 1-methylethyl (isopropyl), n-butyl or 2-methylpropyl (isobutyl), C₁-C₂-fluoroalkyl, especially trifluoromethyl, C₁-C₂-alkoxy, especially methoxy, di(C₁-C₂-alkyl)amino, especially dimethylamio, C₂-C₃-alkenyl, especially allyl, or C₂-C₃-alkynyl, especially propargyl. More preference is given to compounds of the formula (I) in which R² is hydrogen, methyl, ethyl, n-butyl, allyl or propargyl, hydrogen being most preferred.

R¹ is preferably C₁-C₄-alkyl, C₁-C₄-alkoxy, C₁-C₄- alkylthio, di(C₁-C₄-alkylamino, F, Cl and Br. Especially preferably, R¹ is s chlorine, bromine, fluorine, C₁-C₂-alkyl, especially methyl, C₁-C₂-alkoxy, especially methoxy, C₁-C₂-alkylthio, especially methylthio, or di(C₁-C₂-alkyl)amino, especially dimethylamino.

A preferred embodiment of the present invention relates to compounds of the formula (I), in which the index n is zero. These compounds are also referred to compounds (I.a).

A further preferred embodiment of the present invention relates to compounds of the formula (I), in which the index n is one. These compounds are also referred to compounds (I.b). From among these, particular preference is given to those compounds of the formula (I.b), in which R¹ is chlorine, bromine, fluorine, methyl, methoxy, methylthio, dimethylamino, with chlorine being most preferred.

A further preferred embodiment relates to compounds of the formula (I) in which n is two. These compounds are also referred to compounds (I.c). From among these, particular preference is given to those compounds of the formula (I.c), in which R¹ is, independently, from one another, selected from chlorine, methoxy, methyl and methylthio.

A further preferred embodiment relates to compounds of the formula (I) in which n is three. These compounds are also referred to compounds (I.d). From among these, particular preference is given to those compounds of the formula (I.d), in which R¹ is chlorine, methyl, methoxy or methylthio.

Especially preferred are the compounds of the formulae (I.a.a), (I.b.a) and (I.c.a) 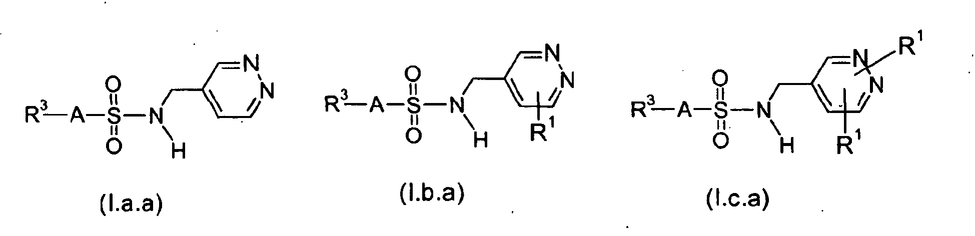 in which R¹, R³ and A are as defined above.

A skilled person will readily understand that the preferences given for R³ and A in connection with compounds of formula (I) also apply for formula (I.a.a) as defined herein. A skilled person will also readily understand that the preferences given for R¹, R³ and A in connection with compounds of formula (I) also apply for formulae (I.b.a) and (I.c.a) as defined herein.

The compounds of the formula (I) according to the invention can be prepared by various routes in analogy to prior art processes known per se for preparing sulfonamide compounds and. advantageously, by the synthesis shown in the following schemes and in the working examples.

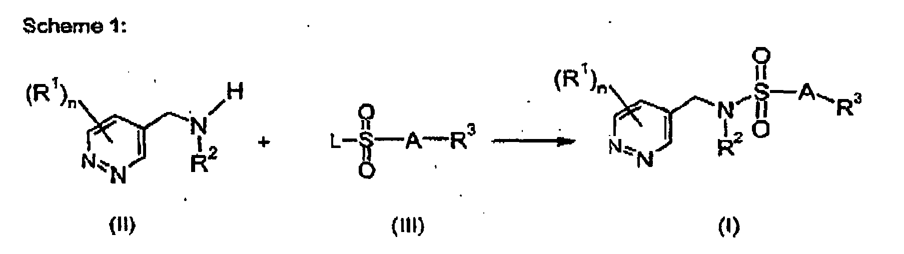

In scheme 1, R¹, R², R³, A and n are as defined above, and L is a leaving group such as hydroxy or halogen, preferably chlorine.

According to the process depicted in scheme 1, a sulfonyl compound (III) is reacted with a pyridazin-ylmethylamine compound (II) to obtain a compound of the formula (I) according to the invention. The reaction of the sulfonyl compound (III) with compound (II) can be performed in accordance with standard methods of organic chemistry, see for example, Lleb. Ann. Chem. P. 641,1990, or WO 2005/033081.

This reaction is usually carried out in an inert organic solvent. Suitable solvents are aliphatic hydrocarbons, such as pentane, hexane, cyclohexane and petroleum ether, aromatic hydrocarbons, such as toluene, o-, m- and p-xylene, halogenated hydrocarbons, such as dichloramethane, trichoromethane and chlorobenzene, ethers, such as diethyl ether, diisopropyl ether, tert.-butyl methyl ether, dioxane, anisole and tetrahydrofuran, nitriles, such as acetonitrils and propionitrile, ketones, such as acetone, methyl ethyl ketone, diethyl ketone and tert-butyl methyl ketone, and also dimethyl sulfoxide, dimethylformamide and dimethylacetamide, preferably tetrahydrofuran, methyl tert.-butylether, dichloromethane, trichloromethane, acetonitrile, toluene or dimethylformamide. It is also possible to use mixtures of the solvents mentioned.

The reaction is carried out in the presence of a base. Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to triethylamine, pyridine, triethylamine, diisopropylethylamine (Hünig base) and potassium carbonate.

The bases are generally employed in catalytic amounts; however, they can also be employed in equimolar amounts, in excess or, if appropriate, as solvent. The excess of base is typically 0.5 to 5 molar equivalents relative to 1 mole of compounds (II).

Generally, the reaction is carried out at temperatures of from -30°C to 120°C, preferably from -10°C to 100°C.

The starting materials are generally reacted with one another in equimolar amounts. In terms of yield, it may be adavantageous to use an excess of compound (II), based on compound (III).

Compounds of the formula (I), wherein R³ is optionally substituted phenyl or 5- or 6-membered heteroaryl and A is optionally substituted phenylene or optionally substituted 5-or 6-membered heteroarenediyl can be prepared, for example, according to the process shown in scheme 2.

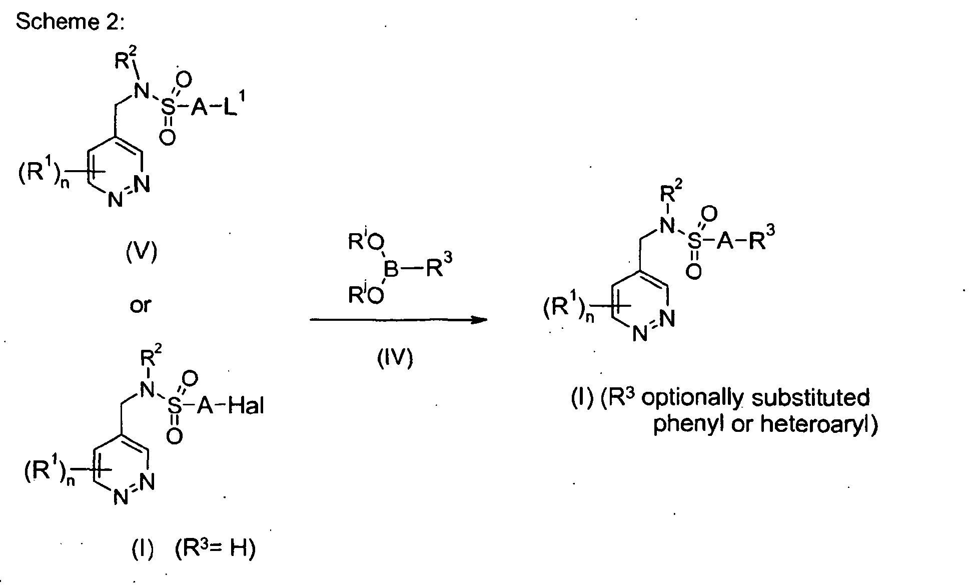

In scheme 2, R¹, R² and n are as defined above, Hal is halogen, especially chlorine, bromine or iodine, most preferably chlorine or bromine halogen, in particular bromine. Rⁱ and R^{j} are each independently hydrogen or C₁-C₄-alkyl, or Rⁱ and R^{j} together form an 1,2-ethylene or 1,2-propylene moiety the carbon atoms of which may be unsubstituted or may all or in part be substituted by methyl groups, and L¹ is a suitable leaving group. Suitable leaving groups L¹ are alkylcarbonylate, benzoate, alkylsulfonate, haloalkylsulfonate or arylsulfonate.

According to scheme 2, compounds of formula (I) wherein R³ is optionally substituted phenyl or 5- or 6-membered heteroaryl and A is optionally substituted phenylene or optionally substituted 5-or 6-membered heteroarenediyl can be prepared by treating a compound of formula (V) with a boronic acid derivative of the formula (IV) under the conditions of a Suzuki coupling. Instead of using a compound of formula (V), it is also possible to use a compound of formula (I), wherein A carries a halogen atom and R³ is hydrogen.

The reaction is usually carried out in the presence of a catalyst, in particular a palladium catalyst, such as for example described in the following literature: Synth. Commun. Vol. 11, p. 513 (1981); Acc. Chem. Res. Vol. 15, pp. 178-184 (1982); Chem. Rev. Vol. 95, pp. 2457-2483 (1995); Organic Letters Vol. 6 (16), p. 2808 (2004); "Metal catalyzed cross coupling reactions", 2nd Edition, Wiley, VCH 2005 (Eds. De Meijere, Diederich); "Handbook of organopalladium chemistry for organic synthesis" (Eds Negishi), Wiley, Interscience, New York, 2002; "Handbook of functionalized organometallics", (Ed. P. Knochel), Wiley, VCH, 2005.

Suitable catalysts are in tetrakis(triphenylphosphine)palladium(0); bis(triphenylphosphine)palladium(II) chloride; bis(acetonitrile)palladium(II) chloride; [1,1'-bis(diphenylphosphino)ferrocene]-palladium(II) chloride/methylene chloride (1:1) complex; bis[bis-(1,2-diphenylphosphino)ethane]palladium(0); bis(bis-(1,2-diphenylphosphino)butane]-palladium(II) chloride; palladium(II) acetate; palladium(II) chloride; and palladium(II) acetate/tri-o-tolylphosphine complex or mixtures of phosphines and Pd salts or phosphines and Pd-complexes e.g. dibenzylideneacetone-palladium and tritertbutylphosphine (or its tetrafluoroborate), tris cyclohexylphosphine; or a polymer-bound Pd-triphenylphosphine catalyst system.

The Suzuki coupling of a compound of the formula (V) and (I), respectively, is carried out in the presence of a base. Suitable bases are, in general, inorganic compounds, such as alkali metal and alkaline earth metal oxides, such as lithium oxide, sodium oxide, calcium oxide and magnesium oxide, alkali metal and alkaline earth metal carbonates, such as lithium carbonate, sodium carbonate, potassium carbonate, caesium carbonate and calcium carbonate, and also alkali metal bicarbonates, such as sodium bicarbonate, alkali metal and alkaline earth metal alkoxides, such as sodium methoxide, sodium ethoxide, potassium ethoxide and potassium tert.-butoxide, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to bases such as sodium carbonate, potassium carbonate, caesium carbonate, triethylamine and sodium bicarbonate.

The base is used in a 1:1 to 1:10; preferably a 1:1.5 to 5 molar ratio relative to 1 mole of compounds (V) and (I), respectively, the boronic acid is used in a 1:1 to 1: 5 ratio, preferably a 1:1 to 1:2.5 molar ratio relative to 1 mole of compounds (V) and (I), respectively. In some cases it may be benefical for easy purification to use the boronic acid in a substoechiometric amount of from 0.7:1 to 0.99:1, per 1 mole of compounds (V) and (I), respectively.

The reaction is usually carried out in an inert organic organic solvent. Suitable solvents are aliphatic hydrocarbons, such as pentane, hexane, cyclohexane and petroleum ether, aromatic hydrocarbons, such as toluene, o-, m- and p-xylene, ethers, such as diisopropyl ether, tert.-butyl methyl ether, dioxane, anisole and tetrahydrofuran and dimethoxyethane, ketones, such as acetone, methyl ethyl ketone, diethyl ketone and tert.-butyl methyl ketone, and also dimethyl sulfoxide, dimethylformamide and dimethylacetamide, particularly preferably ethers, such as tetrahydrofuran, dioxane and dimethoxyethane. It is also possible to use mixtures of the solvents mentioned, or mixtures with water.

The reaction is usually carried out at temperatures of from 20°C to 180°C, preferably from 40°C to 120°C.

After completion of the reaction, the compounds of formula (I) with R³ being different from hydrogen can be isolated by employing conventional methods such as adding the reaction mixture to water, extracting with an organic solvent, concentrating the extract and the like. The isolated compounds (I) can be purified by a technique such as chromatography, recrystallization and the like, if necessary.

It is also possible to add a scavenger to the reaction mixtures to remove byproducts or unreacted starting materials by binding to those and simple filtration. For details see "Synthesis and purification catalog", Argonaut, 2003 and literature cited therein.

Boronic acids or esters (IV) are commercially available or can be prepared according to "Science of Synthesis" Vol. 6, Thieme, 2005; WO 02/042275; Synlett 2003, (8) p.1204; J. Org. Chem., 2003,68, p. 3729, Synthesis, 2000, p.442, J. Org. Chem., 1995, 60, p. 750; or "Handbook of functionalized organometallics", (Ed. P. Knochel), Wiley, VCH, 2005.

Compounds of the formula (V) can be obtained by analogy to the method described in Scheme 1.

Pyridazin-4-ylmethylamine compounds (II) are known from the literature, or they can be prepared, for example, as depicted in schemes 3, 4 or 5 or in the working examples. A route to compounds (II), wherein n = 0 is illustrated in scheme 3 below.

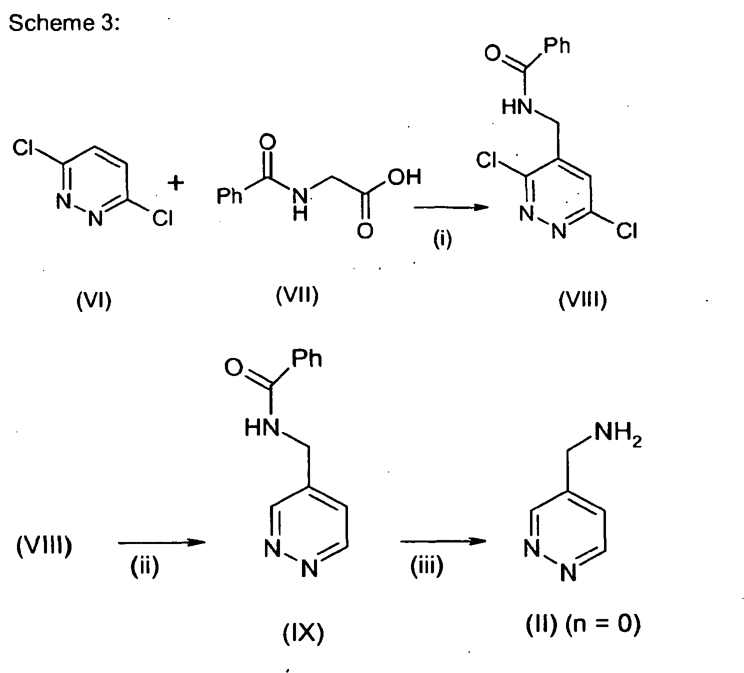

In scheme 3, Ph means phenyl (C₆H₅).

According to scheme 3, 3,6-dichloropyridazine (VI) is treated with hippuric acid (VII) (N-benzoylglycine) in the presence of ammonium peroxydisulfate and silver(I) nitrate in an acidic medium to give the benzamide (VIII) in stage (i). This reaction is known as Minisci radical alkylation, see Org. Lett., 5 (23), 4497 -4499, 2003. A suitable acidic medium is sulphuric acid, a C₁-C₄-alkanoic acid or C₂-C₆-haloalkanoic acid, especially a C₂-C₄-fluoroalkanoic acid such as trifluoroacetic acid, preferably in a mixture with water. This reaction is conveniently carried out at temperatures of from 20°C to 100°C, preferably from 50°C to 100°C. Alternatively, the amide (VIII) can be obtained by treating 3,6-dichloropyridazine (VI) with hippuric acid (VII) in the presence of ammonium peroxydisulfate and silver(I)-carbonate or silver(I)-oxide in a basic medium.

The hippuric acid (VII) is usually used in a molar ratio of 1:1 to 5:1, preferably 1.2:1 to 2.5:1, relative to 1 mole of dichloropyridazine (VI). Silver(I) is usually used in a substoechiometric amount of from 0.05:1 to 0.2:1, per mole of dichloropyridazine (VI). The peroxydisulfate is in general used in a 1:1 to 2:1 ratio, preferably a 1:1 to 1.5:1 molar ratio relative to 1 mole of hippuric acid (VII).

Step (ii) is a reductive dehalogenation with H₂/Pd-C which leads to the N-(pyridazin-4-ylmethyl)benzamide (IX). It may be advantageous to carry out the reductive dehalogenation in the presence of a base. Suitable bases are in general, inorganic compounds, such as alkali metal and alkaline earth metal oxides, such as sodium oxide, and calcium oxide, alkali metal and alkaline earth metal carbonates, such as sodium carbonate, potassium carbonate, and calcium carbonate, and also alkali metal bicarbonates, such as sodium bicarbonate, moreover organic bases, for example tertiary amines, such as trimethylamine, triethylamine, and N-methylpiperidine, pyridine, substituted pyridines, such as collidine, lutidine and 4-dimethylaminopyridine, and also bicyclic amines. Particular preference is given to triethylamine.

Compound (IX), obtained in step ii), is then in step iiii) converted by acid-catalyzed amide hydrolyse into the amine compound (II). Examples of catalysts are hydrogen halides such as hydrogen chloride, or hydrogen bromide, sulphuric acid and the like, with hydrogen chloride being preferred. The hydrolysis is usually carried out at temperatures of from room temperature to the boiling point of the solvent. The amine (II) is usually obtained as salt, preferably as dihydrochloride.

3,6-Dichloropyridazine (VI) and hippuric acid (VII) are commercially available.

Pyridazinylmethylamine compounds (II) carrying a substituent R¹ in the 3-position can be prepared e.g. by the route outlined in scheme 4 below.

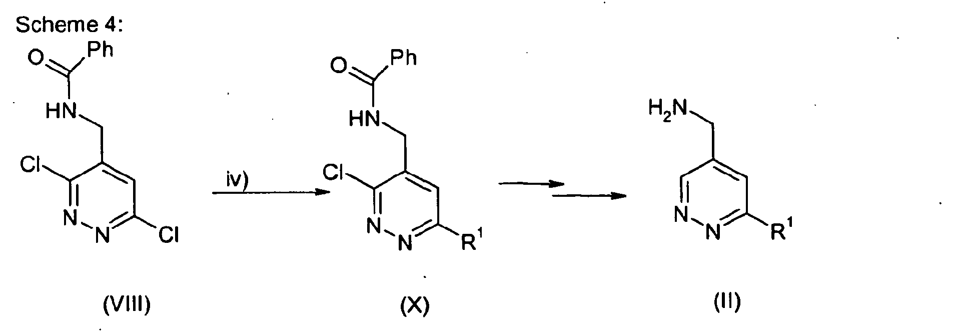

In scheme 4, Ph means phenyl (C₆H₅). R¹ is alkyl, haloalkyl, alkenyl, alkynyl, cycloalkyl, alkylcycloalkyl, alkoxy, haloalkoxy, alkylthio, haloalkylthio or dialkylamino.

In a first step iv), the dichloropyridazine compound (VIII) is reacted with a nucleophile for example an alkoxide, a haloalkoxide, a thiolate or a halothiolate to give a compound of formula (X), wherein R¹ is alkoxy, haloalkoxy, alkylthio, haloalkylthio under reaction conditions known per se, as known for example from Journal of Agricultural and Food Chemistry, 38(2), 508-14, 1990. The reaction is effected advantageously in an inert solvent. Suitable solvents comprise ethers such as dioxane, diethyl ether, methyl tert-butyl ether and preferably tetrahydrofuran, halogenated hydrocarbons such as dichloromethane or dichloroethane, aromatic hydrocarbons such as toluene, and mixtures thereof.

Compounds of the formula (X) in which R¹ is alkyl, haloalkyl, alkenyl, alkynyl, cycloalkyl or alkyl-cycloalkyl can advantageously be prepared by reacting the compound (VIII) with organometallic compounds R^{1a}-Mt where R^{1a} is alkyl, haloalkyl, alkenyl, alkynyl, cycloalkyl or alkyl-cycloalkyl and Mt is lithium, magnesium or zinc. The reaction is effected preferably in the presence of catalytic or, in particular, at least equimolar amounts of transition metal salts and/or compounds, in particular in the presence of Cu salts such as Cu(I) halides and especially Cu(I) iodide. The reaction is effected generally in an inert organic solvent, such as ether, e.g. tetrahydrofuran, an aliphatic or cycloaliphatic hydrocarbon such as hexane, cyclohexane and the like, an aromatic hydrocarbon such as toluene, or in a mixture of these solvents. The temperatures required for this purpose are in the range of from -100 to +100°C and especially in the range from -80°C to +40°C. Alternatively, the introduction of R¹ being alkyl, haloalkyl, alkenyl, alkynyl, cycloalkyl or alkyl-cycloalkyl can be achieved by palladium-catalysed cross coupling between a suitable organoboronic acid or organic boronic ester and the dichloropyridazine compound (VIII) in the sense of a Suzuki coupling. With respect to the reaction conditions for the Suzuki coupling, reference is made to Scheme 2.

The compound (X) is subsequently, analogously to the methods illustrated in Scheme 3, steps ii) and iii) converted into the pyridazin-5-ylmethylamine compound (II) carrying a substituent R¹ in the 3-position.

A pyridazin-4-ylmethylamine compound (II) carrying a substituent R¹ in the 3-position and also a substituent R¹ in the 6-position, wherein each R¹ is different from halogen can be prepared e.g. by the route outlined in scheme 5 below.

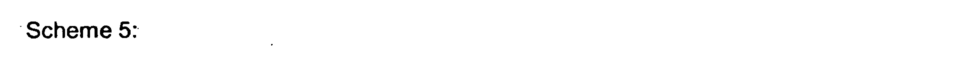

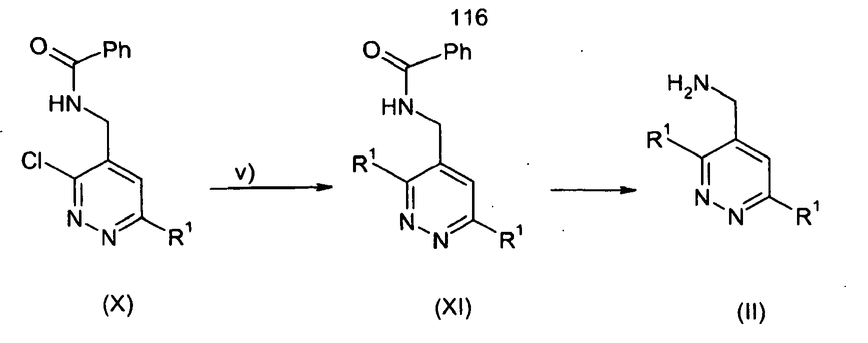

In scheme 5, Ph means phenyl (C₆H₅). R¹ is, independently of one another, alkyl, haloalkyl, alkenyl, alkynyl, cycloalkyl, alkylcycloalkyl, alkoxy, haloalkoxy, alkylthio, haloalkylthio or dialkylamino.

The conversion of the chloropyridazine compound (X) into the pyridazine compound (XI) in step v) is achieved by standard methods of organic chemistry, for example in analogy to the methods illustrated in Scheme 4, step iv). The pyridazine compound (XI) is subsequently, analogously to the methods illustrated in Scheme 3, step iii) converted into the pyridazin-4-ylmethylamine compounds (II).

A pyridazin-4-ylmethylamine compound (II) carrying a substituent R¹ in the 3-position and can be prepared e.g. by the route outlined in scheme 6 below.

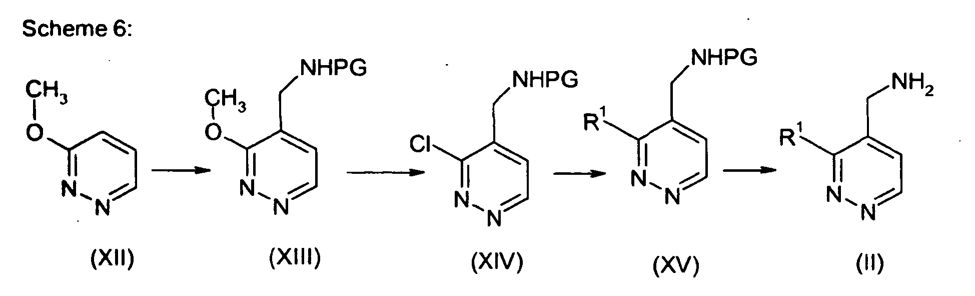

In scheme 6, R¹ is as defined above and PG means an amino protecting group, for example benzyloxycarbonyl or tert-butyloxycarbonyl.

The conversion of the 3-methoxypyridazine compound (XII) into the N-protected pyridazin-4-ylmethylamine compound (XIII) is achieved in a reaction sequence comprising the following steps: first, compound (XII) is treated with a metallation reagent such as 2,2,6,6-tetramethylpiperidine lithium in an inert solvent, for example ether such as tetrahydrofuran; for further details, reference is made to Bull. Korean Chem. Soc. 1996, Vol. 17, no. 9, pages 868-869. The resulting lithiated compound is subsequently reacted with N,N-dimethylformamide to give, after hydrolysis, 3-methoxypyridazine-carbaldehyde. Said aldehyde is treated with hydroxylamine to give the corresponding oxime. Hydrogenation of said oxime compound in the presence of a catalyst, e.g. palladium-on-carbon or Raney nickel, gives a primary amine, whose amino group is, subsequently, protected in order to yield a compound (XIII). Suitable amino-protecting groups are standard amino-protecting groups, such as benzyloxycarbonyl or tert-butyloxycarbonyl. 3-Methoxy-pyridazin-4-ylmethylamine is treated, for example with benzylchloroformate or di-tert-butyl dicarbonate to give the amino-protected pyridazine compound (XIII).

Treatment of (XIII) with trimethylsilyl iodide followed by treatment with phosphoryl chloride affords a 3-chloropyridazine compound (XIV). Compound (XIV) is then reacted with a nucleophil, for example a Grignard-reagent, an organolithium compound, an organoboron compound, an alkoxide, a haloalkoxide, a thiolate or a halothiolate by the methods mentioned in Scheme 5, step v) to give the pyridazine compound (XV). Deprotection of compound (XV) in analogy to the methods mentioned in Scheme 3, step iii) gives the compound of formula (II).

Sulfonyl compounds (III) are commercially available or can be obtained according to procedures known in the art. For example, if A is an optionally substituted thiophenediyl radical, the sulfonyl compound (III) in which L is chlorine can be prepared from the respective halide (XVI) by treatment with alkylmagnesium halogenide such as (CH₃)₂CH-MgCl, SO₂ and SO₂Cl₂ as shown in scheme 7.

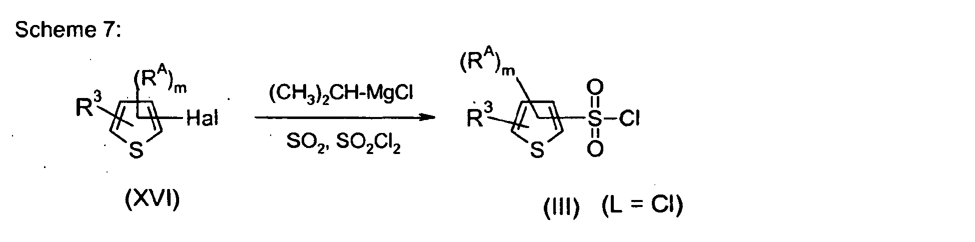

In scheme 7, R³ and R^{A} are as defined above, m is 0, 1, or 2, and Hal means halogen.

Pyridine-SO₃ or dioxane-SO₃ complex converts optionally substituted furan into optionally substituted furan-2-sulfonic acid (III).

Sulfonation of optionally substituted pyrrole with sulfur trioxide-pyridine complex affords mainly the 3-sulfonated pyrroles of formula III, in which A is an optionally substituted pyrrolediyl and L is OH [Mizuno, A. et. al., Tetrahedron Lett. 2000, 41, 6605.]. Sulfonation of optionally substituted imidazole and pyrazole, respectively, with oleum under heating yields optionally substituted imidazole-4-sulfonic acid and optionally substituted pyrrazole-4-sulfonic acid.

Sulfonation of optionally substituted isothiazole and isoxazole, respectively, with oleum takes place at the 4-position, provided that this position is unsubstituted to yield the corresponding sulfonated isothiazole and isoxazole, respectively. Sulfonation of optionally substituted thiazole with oleum at 250°C in the presence of mercury(II)-acetate occurs at the 5-position to yield the corresponding sulfonated thiazole.

Pyridine-2-sulfonic acid, pyridine-3-sulfonic acid and pyridine-4-sulfonic acid are commerically available. For example, sulfonation of pyridine with oleum at 250°C in the presence of Hg(II) as catalyst yields pyridine-3-sulfonic acid. Heating of pyridine-3-sulfonic acid at 360°C or sulfonation of pyridine at 360°C affords the pyridine-4-sulfonic acid. Pyridine-4-sulfonic acid can also be prepared starting from pyridine and thionyl chloride to afford N-(4-pyridyl)pyridinium chloride hydrochloride followed by the treatment of N-(4-pyridyl)pyridinium chloride hydrochloride with sodium sulfite heptahydrate to yield sodium 4-pyridine sulfonate. The treatment of sodium 4-pyridine sulfonate with an HCl affords pyridine-4-sulfonic acid [cf. Organic Syntheses, Coll. Vol. 5, p.977 (1973); Vol. 43, p.97 (1963)].

Compounds of the formula (III), wherein A is optionally substituted phenylene are know from prior art cited in the introductory part.

Compounds of the formula (I) with R² = H can be converted by conventional processes such as alkylation. Examples of suitable alkylating agents include alkyl halides, such as alkyl chloride, alkyl bromide or alkyl iodide, examples being methyl chloride, methyl bromide or methyl iodide, or dialkyl sulfates such as dimethyl sulfate or diethyl sulfate. The reaction with the alkylating agent is carried out advantageously in the presence of a solvent. Solvents used for these reactions are - depending on temperature range-aliphatic, cycloaliphatic or aromatic hydrocarbons such as hexane, cyclohexane, toluene, xylene, chlorinated aliphatic and aromatic hydrocarbons such as dichloromethane, chlorobenzene, open-chain dialkyl ethers such as diethyl ether, di-n-propyl ether, methyl tert-butyl ether, cyclic ethers such as tetrahydrofuran, 1,4-dioxane, glycol ethers such as dimethyl glycol ether, or mixtures of these solvents.

The N-oxides may be prepared from the compounds (I) according to conventional oxidation methods, for example by treating compounds (I) with an organic peracid such as metachloroperbenzoic acid [Journal of Medicinal Chemistry 38(11), 1892-903 (1995), WO 03/64572]; or with inorganic oxidizing agents such as hydrogen peroxide [cf. Journal of Heterocyclic Chemistry 18(7), 1305-8 (1981)] or oxone [cf. Journal of the American Chemical Society 123(25), 5962-5973 (2001)]. The oxidation may lead to pure mono-N-oxides or to a mixture of different N-oxides, which can be separated by conventional methods such as chromatography.

If individual compounds (I) cannot be obtained by the routes described above, they can be prepared by derivatization of other compounds I.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus or pest to be controlled.

The compounds (I) are suitable as fungicides. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). Some are systemically effective and they can be used in crop protection as foliar fungicides, fungicides for seed dressing and soil fungicides.

They are particularly important in the control of a multitude of fungi on various cultivated plants, such as wheat, rye, barley, oats, rice, corn, lawns, bananas, cotton, soybeans, coffee, sugar cane, grapevines, fruit and ornamental plants, and vegetables, such as cucumbers, beans, tomatoes, potatoes and cucurbits, and on the seeds of these plants.

They are especially suitable for controlling the following plant diseases:
- Alternaria species on vegetables, rape, sugar beets, fruit and rice (e.g. A. solani or A. alternata on potatoes and tomatoes),
- Aphanomyces species on sugar beets and vegetables,
- Ascochyta species on cereals and vegetables,
- Bipolaris and Drechslera species on corn, cereals, rice and lawns (e.g. D. maydis on corn),
- Blumeria graminis (powdery mildew) on cereals,
- Botrytis cinerea (gray mold) on strawberries, vegetables, ornamental plants and grapevines,
- Bremia lactucae on lettuce,
- Cerospora species on corn, soybeans, rice and sugar beets,
- Cochliobolus species on corn, cereals, rice (e.g. Cochliobolus sativus on cereals, Cochliobolus miyabeanus on rice),
- Colletotricum species on soybeans and cotton,
- Drechslera species, Pyrenophora species on corn, cereals, rice and lawns (e.g. D. teres on barley or D. tritici-repentis on wheat),
- Esca on grapevines, caused by Phaeoacremonium chlamydosporium, Ph. Aleophilum and Formitipora punctata (syn. Phellinus punctatus),
- Elsinoe ampelina on grapevines,
- Exserohilum species on corn,
- Erysiphe cichoracearum and Sphaerotheca fuliginea on cucurbits,
- Fusarium and Verticillium species on various plants (e.g. F. graminearum or F. culmorum on cereals or F. oxysporum on various plants, e.g. tomatoes),
- Gaeumanomyces graminis on cereals,
- Gibberella species on cereals and rice (e.g. Gibberella fujikuroi on rice),
- Glomerella cingulata on grapevines and other plants,
- Grainstaining complex on rice,
- Guignardia budwelli on grapevines,
- Helminthosporium species on corn and rice,
- Isariopsis clavispora on grapevines,
- Michrodochium nivale on cereals,
- Mycosphaerella species on cereals, bananas and peanuts (e.g. M. graminicola on wheat or M. fijiesis on bananas),
- Peronospora species on cabbage and onion plants (e.g. P. brassicae on cabbage or P. destructor on onions),
- Phakopsara pachyrhizi and Phakopsara meibomiae on soybeans,
- Phomopsis species on soybeans and sun flowers, P. viticola on grapevines,
- Phytophthora infestans on potatoes and tomatoes,
- Phytophthora species on various plants (e.g. P. capsici on paprika),
- Plasmopara viticola on grapevines,
- Podosphaera leucotricha on apples,
- Pseudocercosporella herpotrichoides on cereals,
- Pseudoperonospora on various plants (e.g. P. cubensis on cocumber or P. humili on hops),
- Pseudopezicula tracheiphilai on grapevines,
- Puccinia species on various plants (e.g. P. triticina , P. striformins, P. hordei or P. graminis on cereals or P. asparagi on asparagus),
- Pyricularia oryzae, Corticium sasakii , Sarocladium oryzae, S.attenuatum, Entyloma oryzae on rice,
- Pyricularia grisea on lawns and cereals,
- Pythium spp. on lawns, rice, corn, cotton, rape, sun flowers, sugar beets, vegetables and other plants (e.g. P. ultiumum on various plants, P. aphanidermatum on lawns),
- Rhizoctonia species on cotton, rice, potatoes, lawns, corn, rape, sugar beets, vegetables and on various plants (e.g. R. solani on beets and various plants),
- Rhynchosporium secalis on barley, rye and triticale,
- Sclerotinia species on rape and sun flowers,
- Septoria tritici and Stagonospora nodorum on wheat,
- Erysiphe (syn. Uncinula) necator on grapevines,
- Setospaeria species on corn and lawns,
- Sphacelotheca reilinia on corn,
- Thievaliopsis species on soybeans and cotton,
- Tilletia species on cereals,
- Ustilago species on cereals, corn and sugar cane (e.g. U. maydis on corn),
- Venturia species (scab) on apples and pears (e.g. V. inaequalis on apples).

The compounds (I) are also suitable for controlling harmful fungi in the protection of materials (e.g. wood, paper, paint dispersions, fiber or fabrics) and in the protection of stored products. As to the protection of wood, the following harmful fungi are worthy of note: Ascomycetes such as Ophiostoma spp., Ceratocystis spp., Aureobasidium pullulans, Sclerophoma spp., Chaetomium spp., Humicola spp., Petriella spp., Trichurus spp.; Basidiomycetes such as Coniophora spp., Coriolus spp., Gloeophyllum spp., Lentinus spp., Pleurotus spp., Poria spp., Serpula spp. and Tyromyces spp., Deuteromycetes such as Aspergillus spp., Cladosporium spp., Penicillium spp., Trichoderma spp., Alternaria spp., Paecilomyces spp. and Zygomycetes such as Mucor spp., and in addition in the protection of stored products the following yeast fungi are worthy of note: Candida spp. and Saccharomyces cerevisae.

The compounds (I) are employed by treating the fungi or the plants, seeds, materials or the soil to be protected from fungal attack with a fungicidally effective amount of the active compounds. The application can be carried out both before and after the infection of the materials, plants or seeds by the fungi.

The fungicidal compositions generally comprise between 0.1 and 95%, preferably between 0.5 and 90%, by weight of active compound. The active compounds are employed in a purity of from 90% to 100%, preferably from 95% to 100% (according to NMR spectrum).

When employed in plant protection, the amounts applied are, depending on the kind of effect desired, between 0.01 and 2.0 kg of active compound per ha.

In seed treatment, for example by dusting, coating or drenching seed, amounts of active compound of from 0.1 g to 10 kg, frequently 1 to 1000 g, preferably from 5 to 100 g, per 100 kilogram of seed are generally required.

When used in the protection of materials or stored products, the amount of active compound applied depends on the kind of application area and on the desired effect. Amounts customarily applied in the protection of materials are, for example, 0.001 g to 2 kg, preferably 0.005 g to 1 kg, of active compound per cubic meter of treated material.

In addition the compounds of the formula (I) may also be used in cultures which can tolerate insecticidal or fungal attack due to cultivation, including of genetic engineering.

The compounds of the formula (I) are furthermore suitable for controlling pests from the classes of the insects, arachnids and nematodes effectively. They can be used as pesticides in crop protection and in the sectors of hygiene and the protection of stored products and the veterinary sector.

They may act by contact or may be stomach-acting, or have systemic or residual action. Contact action means that the pest is killed by coming into contact with a compound I or with material that releases compound I. Stomach-acting means that the pest is killed if it ingests a pesticidially effective amount of the compound I or material containing a pesticidally effective amount of compound I. Systemic action means that the compound is absorbed into the plant tissues of treated plant and the pest is controlled, if it eats plant tissue or sucks plant-sap.

Compounds (I) are in particular suitable for controlling the following insect pests:
insects from the order of Lepidoptera, for example Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera eridania, Spodoptera frugiperda, Spodoptera littoralis, Spodoptera litura, Thaumatopoea pityocampa, Tortrix viridana, Trichoplusia ni and Zeiraphera canadensis,
from the order of Coleoptera (beetles), for example Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus and Sitophilus granaria,
from the order of Diptera, for example Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata,
Rhagoletis cerasi, Rhagoletis pomonella, Tabanus bovinus, Tipula oleracea and Tipula paludosa,
from the order of Thysanoptera (thrips), e.g. Dichromothrips spp., Frankliniella fusca, Frankliniella occidentalis, Frankliniella tritici, Scirtothrips citri, Thrips oryzae, Thrips palmi and Thrips tabaci,
ants, bees, wasps, sawflies from the order of (Hymenoptera) e.g. Athalia rosae, Atta cephalotes, Atta cephalotes, Atta laevigata, Atta robusta, Atta capiguara, Atta sexdens, Atta texana, Crematogaster spp., Hoplocampa minuta, Hoplocampa testudinea, Monomorium pharaonis, Solenopsis geminata, and Solenopsis invicta, Solenopsis richteri, Solenopsis xyloni, Pogonomyrmex barbatus, Pogonomyrmex californicus, Pheidole megacephala, Dasymutilla occidentalis, Bombus spp. Vespula squamosa, Paravespula vulgaris, Paravespula pennsylvanica, Paravespula germanica, Dolichovespula maculata, Vespa crabro, Polistes rubiginosa, Camponotus floridanus, and Linepithema humile,
from the order of Homoptera, e.g. Acyrthosiphon onobrychis, Adelges laricis, Aphidula nasturtii, Aphis craccivora, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisa tabaci, Bemisa argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, and Viteus vitifolii,
from the order of Isoptera (termites), e.g. Calotermes flavicollis, Heterotermes aureus, Leucotermes flavipes, Reticulitermes flavipes, Reticulitermes virginicus, Reticulitermes lucifugus, aund Termes natalensis, and Coptotermes formosanus,
cockroaches (Blattaria - Blattodea), e.g. Blattella germanica, Blattella asahinae, Periplaneta americana, Periplaneta japonica, Periplaneta brunnea, Periplaneta fuligginosa, Periplaneta australasiae, and Blatta orientalis,
true bugs (Hemiptera), e.g. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis , Thyanta perditor, Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisia argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzus persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand, Viteus vitifolii, Cimex lectularius, Cimex hemipterus, Reduvius senilis, Triatoma spp., and Arilus critatus,
crickets, grasshoppers, locusts from the order of (Orthoptera), e.g. Acheta domestica, Blatta orientalis, Blattella germanica, Calliptamus italicus, Chortoicetes terminifera, Dociostaurus maroccanus, Forficula auricularia, Gryllotalpa gryllotalpa, Hieroglyphus daganensis, Kraussaria angulifera, Locusta migratoria, Locustana pardalina, Melanoplus bivittatus, Melanoplus femur-rubrum, Melanoplus mexicanus, Melanoplus sanguinipes, Melanoplus spretus, Nomadacris septemfasciata, Oedaleus senegalensis, Periplaneta americana, Schistocerca americana, Schistocerca peregrina, Schistocerca gregaria, Stauronotus maroccanus and, Tachycines asynamorus, Tachycines asynamorus, Zonozerus variegatus.

The compounds of the formula (I) and their salts are also useful for controlling arachnids (Arachnoidea), such as acarians (Acarina), e.g. of the families Argasidae, Ixodidae and Sarcoptidae, such as Amblyomma americanum, Amblyomma variegatum, Ambryomma maculatum, Argas persicus, Boophilus annulatus, Boophilus decoloratus, Boophilus microplus, Dermacentor silvarum, Dermacentor andersoni, Dermacentor variabilis, Hyalomma truncatum, Ixodes ricinus, Ixodes rubicundus, Ixodes scapularis, Ixodes holocyclus, Ixodes pacificus, Ornithodorus hermsi, Ornithodorus turicata, Ornithonyssus bacoti, Ornithodorus moubata, Otobius megnini, Dermanyssus gallinae, Psoroptes ovis, Rhipicephalus sanguineus, Rhipicephalus appendiculatus, Rhipicephalus evertsi, Sarcoptes scabiei, and Eriophyidae spp. such as Aculus schlechtendali, Phyllocoptrata oleivora and Eriophyes sheldoni; Tarsonemidae spp. such as Phytonemus pallidus and Polyphagotarsonemus latus; Tenuipalpidae spp. such as Brevipalpus phoenicis; Tetranychidae spp. such as Tetranychus cinnabarinus, Tetranychus kanzawai, Tetranychus pacificus, Tetranychus telarius and Tetranychus urticae, Panonychus ulmi, Panonychus citri, and oligonychus pratensis.and Oligonychus pratensis; Araneida, e.g. Latrodectus mactans, and Loxosceles reclusa,
fleas (Siphonaptera), e.g. Ctenocephalides felis, Ctenocephalides canis, Xenopsylla cheopis, Pulex irritans, Tunga penetrans, and Nosopsyllus fasciatus,
silverfish, firebrat (Thysanura), e.g. Lepisma saccharina and Thermobia domestica,
centipedes (Chilopoda), e.g. Scutigera coleoptrata,
millipedes (Diplopoda), e.g. Narceus spp.,
earwigs (Dermaptera), e.g. forficula auricularia,
lice (Phthiraptera), e.g. Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus and Solenopotes capillatus.

The compounds of the formula (I), their N-oxides and their salts are also useful for controlling nematodes, for example, root gall nematodes, e.g. Meloidogyne hapla, Meloidogyne incognita, Meloidogyne javanica, cyst-forming nematodes, e.g. Globodera rostochiensis, Heterodera avenae, Heterodera glycines, Heterodera schachtii, Heterodera trifolii, stem and leaf nematodes, e.g. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratytenchus curvitatus and Pratylenchus goodeyi. Compounds of the formula (I) are particularly useful for controlling insects of the order Lepidoptera.

In general, the insecticidal compositions comprise from 0.01 to 95% by weight, preferably from 0.1 to 90% by weight, of the active compound. The active compounds are employed in a purity of from 90% to 100%, preferably from 95% to 100% (according to NMR spectrum).

Under outdoor conditions, the active compound application rate for controlling pests is from 0.1 to 2.0, preferably from 0.2 to 1.0, kg/ha.

The compounds (I), their N-oxides and salts can be converted into customary formulations (agricultural formulations), e.g. solutions, emulsions, suspensions, dusts, powders, pastes and granules. The application form depends on the particular intended purpose; in each case, it should ensure a fine and uniform distribution of the compound according to the invention.

The formulations are prepared in a known manner, e.g. by extending the active ingredient with solvents and/or carriers, if desired using emulsifiers and dispersants. Solvents/auxiliaries, which are suitable, are essentially:
- water, aromatic solvents (for example Solvesso^{®} products, xylene), paraffins (for example mineral fractions), alcohols (for example methanol, butanol, pentanol, benzyl alcohol), ketones (for example cyclohexanone, gamma-butyrolactone), pyrrolidones (N-methylpyrrolidone, N-octylpyrrolidone), acetates (glycol diacetate), glycols, fatty acid dimethylamides, fatty acids and fatty acid esters. In principle, solvent mixtures may also be used.
- carriers such as ground natural minerals (e.g. kaolins, clays, talc, chalk) and ground synthetic minerals (e.g. highly disperse silica, silicates); emulsifiers such as nonionic and anionic emulsifiers (e.g. polyoxyethylene fatty alcohol ethers, alkyl-sulfonates and arylsulfonates) and dispersants such as lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalene-sulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenyl ether, ethoxylated isooctylphenol, octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Also anti-freezing agents such as glycerin, ethylene glycol and propylene glycol can be added to the formulation.

Suitable antifoaming agents are, for example, those based on silicone or magnesium stearate.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

Formulations for the treatment of seed may additionally comprise binders and/or gelling agents and, if appropriate, colorants.

Binders may be added to increase the adhesion of the active compounds on the seed after the treatment. Suitable binders are, for example, EO/PO block copolymer surfactants, but also polyvinyl alcohols, polyvinylpyrrolidones, polyacrylates, polymethacrylates, polybutenes, polyisobutylenes, polystyrenes, polyethylenamines, polyethylenamides, polyethylenimines (Lupasol^{®}, Polymin^{®}), polyethers, polyurethanes, polyvinyl acetates, tylose and copolymers of these polymers.

A suitable gelling agent is, for example, carrageen (Satiagel^{®}).

In general, the formulations comprise from 0.01 to 95% by weight, preferably from 0.1 to 90% by weight, of the active compound. The active compounds are employed in a purity of from 90% to 100%, preferably from 95% to 100% (according to NMR spectrum).

The concentrations of active compound in the ready-for-use preparations can be varied within relatively wide ranges. In general, they are between 0.0001 and 10%, preferably between 0.01 and 1%.

The active compounds can also be used with great success in the ultra-low volume (ULV) process, it being possible to apply formulations with more than 95% by weight of active compound or even the active compound without additives.

For the treatment of seed, the formulations in question give, after two-to-tenfold dilution, active compound concentrations of from 0.01 to 60% by weight, preferably from 0.1 to 40% by weight, in the ready-to-use preparations.

The following are examples of formulations: 1. Products for dilution with water

### A) Water-soluble concentrates (SL, LS)

10 parts by weight of a compound I according to the invention are dissolved in 90 parts by weight of water or in a water-soluble solvent. As an alternative, wetting agents or other auxiliaries are added. The active compound dissolves upon dilution with water. In this way, a formulation having a content of 10% by weight of active compound is obtained.

### B) Dispersible concentrates (DC)

20 parts by weight of a compound I according to the invention are dissolved in 70 parts by weight of cyclohexanone with addition of 10 parts by weight of a dispersant, for example polyvinylpyrrolidone. Dilution with water gives a dispersion. The active compound content is 20% by weight.

### C) Emulsifiable concentrates (EC)

15 parts by weight of a compound I according to the invention are dissolved in 75 parts by weight of xylene with addition of calcium dodecylbenzenesulfonate and castor oil ethoxylate (in each case 5 parts by weight). Dilution with water gives an emulsion. The formulation has an active compound content of 15% by weight.

### D) Emulsions (EW, EO, ES)

25 parts by weight of a compound I according to the invention are dissolved in 35 parts by weight of xylene with addition of calcium dodecylbenzenesulfonate and castor oil ethoxylate (in each case 5 parts by weight). This mixture is introduced into 30 parts by weight of water by means of an emulsifying machine (Ultraturrax) and made into a homogeneous emulsion. Dilution with water gives an emulsion. The formulation has an active compound content of 25% by weight.

### E) Suspensions (SC, OD, FS)

In an agitated ball mill, 20 parts by weight of a compound I according to the invention are comminuted with addition of 10 parts by weight of dispersants and wetting agents and 70 parts by weight of water or an organic solvent to give a fine active compound suspension. Dilution with water gives a stable suspension of the active compound. The active compound content in the formulation is 20% by weight.

### F) Water-dispersible granules and water-soluble granules (WG, SG)

50 parts by weight of a compound I according to the invention are ground finely with addition of 50 parts by weight of dispersants and wetting agents and prepared as water-dispersible or water-soluble granules by means of technical appliances (for example extrusion, spray tower, fluidized bed). Dilution with water gives a stable dispersion or solution of the active compound. The formulation has an active compound content of 50% by weight.

### G) Water-dispersible powders and water-soluble powders (WP, SP, SS, WS)

75 parts by weight of a compound I according to the invention are ground in a rotor-stator mill with addition of 25 parts by weight of dispersants, wetting agents and silica gel. Dilution with water gives a stable dispersion or solution of the active compound. The active compound content of the formulation is 75% by weight.

### H) Gel (GF)

In an agitated ball mill, 20 parts by weight of a compound I according to the invention are comminuted with addition of 10 parts by weight of dispersants, 1 part by weight of a gelling agent wetters and 70 parts by weight of water or of an organic solvent to give a fine suspension of the active compound. Dilution with water-gives a stable suspension of the active compound, whereby a formulation with 20% (w/w) of active compound is obtained.

### 2. Products to be applied undiluted

### J) Dustable powders (DP, DS)

5 parts by weight of a compound I according to the invention are ground finely and mixed intimately with 95 parts by weight of finely divided kaolin. This gives a dustable product having an active compound content of 5% by weight.

### K) Granules (GR, FG, GG, MG)

0.5 part by weight of a compound I according to the invention is ground finely and associated with 99.5 parts by weight of carriers. Current methods are extrusion, spray-drying or the fluidized bed. This gives granules to be applied undiluted having an active compound content of 0.5% by weight.

### L) ULV solutions (UL)

10 parts by weight of a compound I according to the invention are dissolved in 90 parts by weight of an organic solvent, for example xylene. This gives a product to be applied undiluted having an active compound content of 10% by weight.

The active ingredients can be used as such, in the form of their formulations or the use forms prepared therefrom, eg. in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; it is intended to ensure in each case the finest possible distribution of the active ingredients according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.0001 to 10%, preferably from 0.001 to 1%.

The active ingredients may also be used successfully in the ultra-low-volume process (ULV), it being possible to apply formulations comprising over 95% by weight of active ingredient, or even to apply the active ingredient without additives.

Various types of oils, wetters, adjuvants, herbicides, fungicides, other pesticides, or bactericides may be added to the active compounds, if appropriate not until immediately prior to use (tank mix). These agents can be admixed with the agents according to the invention in a weight ratio of 1:10 to 10:1.

The compositions according to the invention can, in the use form as fungicides, also be present together with other active compounds, e.g. with herbicides, insecticides, growth regulators, fungicides or else with fertilizers. Mixing the compounds (I) or the compositions comprising them in the use form as fungicides with other fungicides results in many cases in an expansion of the fungicidal spectrum of activity being obtained.

The following list of fungicides, in conjunction with which the compounds according to the invention can be used, is intended to illustrate the possible combinations but does not limit them:
- acylalanines, such as benalaxyl, metalaxyl, ofurace or oxadixyl,
- amine derivatives, such as aldimorph, dodine, dodemorph, fenpropimorph, fenpropidin, guazatine, iminoctadine, spiroxamine or tridemorph,
- anilinopyrimidines, such as pyrimethanil, mepanipyrim or cyprodinil,
- antibiotics, such as cycloheximide, griseofulvin, kasugamycin, natamycin, polyoxin or streptomycin,
- azoles, such as bitertanol, bromoconazole, cyproconazole, difenoconazole, dinitroconazole, enilconazole, epoxiconazole, fenbuconazole, fluquinconazole, flusilazole, flutriafol, hexaconazole, imazalil, ipconazole, metconazole, myclobutanil, penconazole, propiconazole, prochloraz, prothioconazole, simeconazole, tebuconazole, tetraconazole, triadimefon, triadimenol, triflumizole or triticonazole,
- dicarboximides, such as iprodione, myclozolin, procymidone or vinclozolin,
- dithiocarbamates, such as ferbam, nabam, maneb, mancozeb, metam, metiram, propineb, polycarbamate, thiram, ziram or zineb,
- heterocyclic compounds, such as anilazine, benomyl, boscalid, carbendazim, carboxin, oxycarboxin, cyazofamid, dazomet, dithianon, famoxadone, fenamidone, fenarimol, fuberidazole, flutolanil, furametpyr, isoprothiolane, mepronil, nuarimol, picobenzamide, probenazole, proquinazid, pyrifenox, pyroquilon, quinoxyfen, silthiofam, thiabendazole, thifluzamide, thiophanate-methyl, tiadinil, tricyclazole or triforine,
- copper fungicides, such as Bordeaux mixture, copper acetate, copper oxychloride or basic copper sulfate,
- nitrophenyl derivatives, such as binapacryl, dinocap, dinobuton or nitrophthalisopropyl,
- phenylpyrroles, such as fenpiclonil or fludioxonil,
- sulfur,
- other fungicides, such as acibenzolar-S-methyl, benthiavalicarb, carpropamid, chlorothalonil, cyflufenamid, cymoxanil, diclomezine, diclocymet, diethofencarb, edifenphos, ethaboxam, fenhexamid, fentin acetate, fenoxanil, ferimzone, fluazinam, fosetyl, fosetyl-aluminum, phosphorous acid, iprovalicarb, hexachlorobenzene, metrafenone, pencycuron, penthropyrad, propamocarb, phthalide, toloclofos-methyl, quintozene or zoxamide,
- strobilurins, such as azoxystrobin, dimoxystrobin, enestroburin, fluoxastrobin, kresoxim-methyl, metominostrobin, orysastrobin, picoxystrobin, pyraclostrobin or trifloxystrobin,
- sulfenic acid derivatives, such as captafol, captan, dichlofluanid, folpet or tolylfluanid,
- cinnamides and analogous compounds, such as dimethomorph, flumetover or flumorph.

Compositions of this invention may also contain other active ingredients, for example other pesticides such as insecticides and herbicides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredients.

These agents usually are admixed with the agents according to the invention in a weight ratio of 1:100 to 100:1.

The following list of pesticides together with which the compounds according to the invention can be used, is intended to illustrate the possible combinations, but not to impose any limitation:
A.1. Organo(thio)phosphates: e.g. acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
A.2. Carbamates: e.g. alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
A.3. Pyrethroids: e.g. allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
A.4. Growth regulators: a) chitin synthesis inhibitors: e.g. benzoylureas: chlorfluazuron, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: e.g. halofenozide, methoxyfenozide, tebufenozide, azadirachtin; c) juvenoids: e.g. pyriproxyfen, methoprene, fenoxycarb; d) lipid biosynthesis inhibitors: e.g. spirodiclofen, spiromesifen or spirotetramat;
A.5. Nicotinic receptor agonists/antagonists compounds (nicotinoid insecticides or neonicotinoids): e.g. clothianidin, dinotefuran, imidacloprid, thiamethoxam, nitenpyram, acetamiprid, thiacloprid or the thiazol compound of formula P1 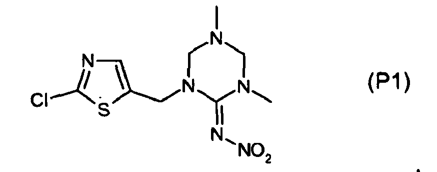
A.6. GABA antagonist compounds: e.g. acetoprole, endosulfan, ethiprole, fipronil, vaniliprole, pyrafluprole, pyriprole, 5-amino-3-(aminothiocarbonyl)-1-(2,6-dichloro-4-trifluoromethylphenyl)-4-(trifluoromethylsulfinyl)-pyrazole;
A.7. Macrocyclic lactone insecticides: abamectin, emamectin, milbemectin, lepimectin, spinosad,
A.8. Mitochondrial complex I electron transport inhibitors (METI I compounds): e.g. fenazaquin, pyridaben, tebufenpyrad, tolfenpyrad, flufenerim;
A.9. Mitochondrial complex II and/or complex III electron transport inhibitors (METI II and III compounds): e.g. acequinocyl, fluacyprim, hydramethylnon;
A.10. Uncoupler compounds: e.g. chlorfenapyr;
A.11. Oxidative phosphorylation inhibitor compounds: cyhexatin, diafenthiuron, fenbutatin oxide, propargite;
A.12. Moulting disruptor compounds: e.g. cyromazine;
A.13. Mixed function oxidase inhibitor compounds: e.g. piperonyl butoxide;
A.14. Sodium channel blocker compounds: e.g. indoxacarb, metaflumizone,
A.15. Various: benclothiaz, bifenazate, cartap, flonicamid, pyridalyl, pymetrozine, sulfur, thiocyclam, flubendiamide, cyenopyrafen, flupyrazofos, cyflumetofen, amidoflumet, compounds of the formula P2: 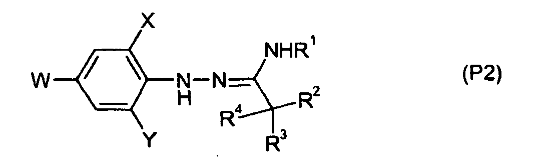 wherein X and Y are each independently halogen, in particular chlorine;
   W is halogen or C₁-C₂-haloalkyl, in particular trifluoromethyl;
   R¹ is C₁-C₆-alkyl, C₂-C₆-alkenyl, C₂-C₆-alkynyl, C₁-C₄-alkoxy-C₁-C₄-alkyl or C₃-C₆-cycloalkyl each of which may be substituted with 1, 2, 3, 4 or 5 halogen atoms; in particular R¹ is methyl or ethyl;
   R² and R³ are C₁-C₆-alkyl, in particular methyl, or may form together with the adjacent carbon atom a C₃-C₆-cycloalkyl moiety, in particular a cyclopropyl moiety, which may carry 1, 2 or 3 halogen atoms, examples including 2,2-dichlorocyclopropyl and 2,2-dibromocyclopropyl; and
   R⁴ is hydrogen or C₁-C₆-alkyl, in particular hydrogen methyl or ethyl;
   anthranilamide compounds of formula P3 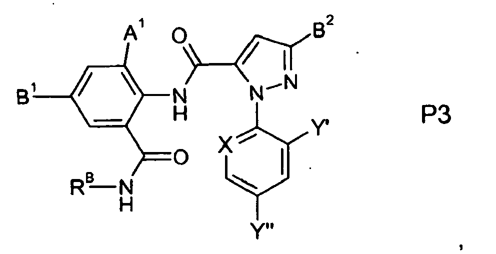 wherein A¹ is CH₃, Cl, Br, I, X is C-H, C-Cl, C-F or N, Y' is F, Cl, or Br, Y" is F, Cl, CF₃, B¹ is hydrogen, Cl, Br, I, CN, B² is Cl, Br, CF₃, OCH₂CF₃, OCF₂H, and R⁸ is hydrogen, CH₃ or CH(CH₃)₂;
   and malononitrile compounds as described in JP 2002/284608, WO 02/89579, WO 02/90320, WO 02/90321, WO 04/06677, WO 04/20399 or JP 2004/99597.

Suitable pesticides compounds also include microorganisms such as Bacillus thuringiensis, Bacillus tenebrionis and Bacillus subtilis.

The aforementioned compositions are particularly useful for protecting plants against infestation of said pests and also for protecting plants against infections of phytopathogenic fungi or to combat these pests/fungi in infested/infected plants.

However, the compounds of formula (I) are also suitable for the treatment of seeds. Application to the seeds is carried out before sowing, either directly on the seeds or after having pregerminated the latter.

Compositions which are useful for seed treatment are e.g.:
- A: Soluble concentrates (SL, LS)
- D: Emulsions (EW, EO, ES)
- E: Suspensions (SC, OD, FS)
- F: Water-dispersible granules and water-soluble granules (WG, SG)
- G: Water-dispersible powders and water-soluble powders (WP, SP, WS)
- H: Dustable powders (DP, DS)

Preferred FS formulations of compounds of formula (I) for seed treatment usually comprise from 0.5 to 80% of the active ingredient, from 0,05 to 5 % of a wetter, from 0.5 to 15 % of a dispersing agent, from 0,1 to 5 % of a thickener, from 5 to 20 % of an anti-freeze agent, from 0,1 to 2 % of an anti-foam agent, from 1 to 20 % of a pigment and/or a dye, from 0 to 15 % of a sticker / adhesion agent, from 0 to 75 % of a filler/vehicle, and from 0,01 to 1 % of a preservative.

Suitable pigments or dyes for seed treatment formulations are pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Stickers / adhesion agents are added to improve the adhesion of the active materials on the seeds after treatment. Suitable adhesives are block copolymers EO/PO surfactants but also polyvinylalcohols, polyvinylpyrrolidones, polyacrylates, polymethacrylates, polybutenes, polyisobutylenes, polystyrene, polyethyleneamines, polyethyleneamides, polyethyleneimines (Lupasol^{®}, Polymin^{®}), polyethers and copolymers derived from these polymers.

For use against ants, termites, wasps, flies, mosquitos, crickets, or cockroaches, compounds of formula (I) are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

Formulations of compounds of formula (I) as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250°C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

The compounds of formula (I) and its respective compositions can also be used in mosquito and fumigating coils, smoke cartridges, vaporizer plates or long-term vaporizers and also in moth papers, moth pads or other heat-independent vaporizer systems.

The compounds of formula (I) and its compositions can be used for protecting non-living material, in particular cellulose-based materials such as wooden materials e.g. trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of formula (I) are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied to the crops or the surrounding soil, or is directly applied to the nest of ants or the like.

In the methods according to the invention the pests are controlled by contacting the target parasite/pest, its food supply, habitat, breeding ground or its locus with a pesticidally effective amount of at least one compounds (I), or the N-oxide or salt thereof, or with a composition, containing a pesticidally effective amount of at least one compound I, or the N-oxide or salt thereof.

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds (I) of the invention can also be applied preventively to places at which occurrence of the pests is expected.

The compounds of formula (I) may be also used to protect growing plants from attack or infestation by pests by contacting the plant with a pesticidally effective amount of compounds of formula (I). As such, "contacting" includes both direct contact (applying the compounds/compositions directly on the pest and/or plant - typically to the foliage, stem or roots of the plant) and indirect contact (applying the compounds/compositions to the locus of the pest and/or plant).

The compounds (I) are employed by treating the fungi, pests or the plants, seeds, materials or the soil to be protected from fungal attack or pesticidal attack with a fungicidally or pesticidally effective amount of at least one active compound I, its N-oxide or salt. The application can be carried out both before and after the infection / infestation of the materials, plants or seeds by the fungi or pest.

When employed in plant protection, the amounts applied are, depending on the kind of effect desired, in the range of 0.1 g to 4000 g per hectare, desirably from 25 g to 600 g per hectare, more desirably from 50 g to 500 g per hectare.

In the treatment of seed, the application rates of the active compounds are generally from 0.001 g to 100 g per kg of seed, preferably from 0.01 g to 50 g per kg of seed, in particular from 0.01 g to 2 g per kg of seed.

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and / or insecticide.

For use in bait compositions, the typical content of active ingredient is from 0.001 weight % to 15 weight %, desirably from 0.001 weight % to 5% weight % of active compound.

For use in spray compositions, the content of active ingredient is from 0.001 to 80 weights %, preferably from 0.01 to 50 weight % and most preferably from 0.01 to 15 weight %.

When used in the protection of materials or stored products, the amount of active compound applied depends on the kind of application area and on the desired effect. Amounts customarily applied in the protection of materials are, for example, 0.001 g to 2 kg, preferably 0.005 g to 1 kg, of active compound per cubic meter of treated material.

Under outdoor conditions, the active compound application rate for controlling pests is from 0.1 to 2.0, preferably from 0.2 to 1.0, kg/ha.

Various types of oils, wetters, adjuvants, herbicides, fungicides, other pesticides, or bactericides may be added to the active compounds, if appropriate not until immediately prior to use (tank mix). These agents can be admixed with the agents according to the invention in a weight ratio of 1:100 to 100:1, preferably 1:10 to 10:1.

Adjuvants which can be used are in particular organic modified polysiloxanes such as Break Thru S 240^{®}; alcohol alkoxylates such as Atplus 245^{®}, Atplus MBA 1303^{®}, Plurafac LF 300^{®} and Lutensol ON 30^{®}; EO/PO block polymers, z. B. Pluronic RPE 2035^{®} and Genapol B^{®}; alcohol ethoxylates such as Lutensol XP 80^{®}; and dioctyl sulfosuccinate sodium such as Leophen RA^{®}.

Compounds of formula (I), their N-oxides and veterinarily acceptable salts as well as the compositions comprising them can also be used for controlling and preventing infestations and infections in animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula (I) and compositions comprising them are suitable for systemic and/or non-systemic control of ecto- and/or endoparasites. They are active against all or some stages of development.

Administration can be carried out both prophylactically and therapeutically. Administration of the active compounds (I)s carried out directly or in the form of suitable preparations, orally, topically/dermally or parenterally.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds, their N-oxides and salts may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Generally it is favorable to apply solid formulations which release compounds of formula (I) in total amounts of 10 mg/kg to 300 mg/kg, preferably 20 mg/kg to 200 mg/kg.

The active compounds can also be used as a mixture with synergists or with other active compounds which act against pathogenic endo- and ectoparasites.

In general, the compounds of formula (I) are applied in parasiticidally effective amount-meaning the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

### Synthesis examples

### Preparation of intermediates:

### I. Preparation of pyridazin-4-ylmethylamine dihydrochloride

### I.1.1 N-[(3,6-Dichloropyridazin-4-yl)methyl]benzamide

A 5-L round-bottomed flask equipped with a mechanical stirrer was charged with 3,6-dichloropyridazine (100 g), hippuric acid (204 g), silver nitrate (11.4 g), water (1.2 L) and trifluoroacetic acid (15.3 g) under nitrogen. The mixture was warmed to 70°C, and a solution of ammonium peroxydisulfate (275 g) in water (400 mL) was quickly added. Doing this, the reaction mixture warmed up to 87-88°C. The reaction mixture was cooled to 70°C and stirred at said temperature for 30 minutes. The reaction mixture was diluted with isopropyl acetate (2 L), cooled to 10°C and made alkaline to pH 9 by addition of a concentrated ammonium hydroxide solution. Doing this, the temperature was always kept below 25°C using an ice-bath. The phases were separated and the aqueous phase was further extracted with isopropyl acetate (3x500 mL). The organic phase was washed with an aqueous solution of sodium hydrogen carbonate (1 M), dried (MgSO₄), filtered and evaporated in vacuo to afford 141 g of the title compound as white solid.
¹H NMR (500 MHz, CDCl₃) δ 7.86 (m, 2H), 7.55 (t, 1H, J = 7.3 Hz), 7.44 (m, 4H), 4.66 (d, 2H, J = 6.0 Hz); ESI-MS m/z 283 [C₁₂H₉Cl₂N₃O + H]⁺.

### I.1.2 N-(Pyridazin-4-ylmethyl)benzamide

A 500-mL reaction vessel was charged with N-[(3,6-dichloropyridazin-4-yl)methyl]benzamide (25 g), triethylamine (22.4 g), palladium on carbon (1.5 g, 10%, 50% H₂O) and 300 mL of ethanol under nitrogen. The reaction vessel was evacuated and flushed with hydrogen. This procedure was repeated three timies. After completion of the reaction, the reaction vessel was evacuated, flushed with nitrogen and the reaction mixture was filtered through a Celite® pad. The pad was washed three times with 300 mL of ethanol. The filtrate was evaporated to afford a yellow, waxy residue. The residue was taken up in 500 mL of dichloromethane and extracted with 500 mL of a saturated aqueous sodium hydrogencarbonate solution. The aqueous phase was extracted with 200 mL of dichloromethane (3x). The organic phase was dried (Na₂SO₄), filtered and evaporated to afford the title compound (18.9 g, 100 %) as yellow solid.
¹H NMR (500 MHz, CDCl₃) δ 9.10 (s, 1H), 9.01 (dd, 1H, J = 7.0, 1.1 Hz), 7.86 (m, 3H), 7.50 (m, 1H), 7.43 (m, 3H), 4.64 (d, 2H, J = 6.0 Hz); ESI-MS m/z 214 [C₁₂H₁₁N₃O + H]⁺.

### I.1.3 Pyridazin-4-ylmethylamine dihydrochloride

N-(Pyridazin-4-ylmethyl)-benzamide (310 g) in 2.5 L of 6N HCl was heated under reflux for 7 h. After cooling to room temperature, the reaction mixture was extracted (3x) with diethyl ether (1 L) and the aqueous phase was evaporated in vacuo to afford the title compound (251 g, 95%) as brown solid.
¹H NMR (500 MHz, DMSO-d₆) δ 11.9 (br s, 1H), 9.64 (dd, 1H, J = 3.0, 1.1 Hz), 9.52 (dd. 1H, J = 5.0, 1.0 Hz), 9.17 (br s, 3H), 8.33 (dd, 1H, J = 5.5, 2.3 Hz), 4.64 (dd, 2H, J = 11.0, 5.5 Hz); ESI-MS m/z 110 [C₅H₇N₃ + H]⁺.

### II. Preparation of sulfonic acid amides (I)

### Example 1: 4-Bromothiophene-2-sulfonic acid-(pyridazin-4-ylmethyl)-amide

A solution of 4-bromothiophene-2-sulfonylchloride (33.3 g, 127 mmol) was passed to an ice-cold mixture of pyridazin-4-ylmethylamine dihydrochloride (23.18 g, 127 mmol) and N,N-diisopropylethylamine (Hünig base) in 1 L of dichloromethane, while the temperature was maintained between 0 and 5°C. The reaction mixture was allowed to warm to room temperature and stirred for 1 h. The reaction mixture was poured onto saturated aqueous sodium hydrogencarbonate solution (1 L). The aqueous phase was extracted with ethyl acetate (2x 1 L). The organic phase was dried (Na₂SO₄), filtered and evaporated to afford the crude product. Chromatography (CH₂Cl₂/CH₃CN/CH₃OH 77 : 20 : 3) afforded the title compound (24.7 g, 58 %) as brown solid of melting point 120- 122 °C.
¹H NMR (300 MHz, CH₃OD) δ 9.13- 9.09 (m, 2H), 7.78 (d, J = 1.53 Hz, 1H), 7.69-7.66 (m, 2H), 7.55 (d, J = 1.62 Hz, 1H), 4.3 (s, 2H); ESI-MS m/z 334 [C₉H₈BrN₃O₂S₂ + H]⁺.

### General procedure for the preparation of compounds (I) prepared by Suzuki coupling

A mixture of a suitable sulfonamide of general formula (I) with R³ is halogen (1.20 mmol), a suitable boronic acid (1.44 mmol) and Pd(PPh₃)₂Cl₂ (0.09 mmol) in 1 mL of a saturated aqueous sodium carbonate solution and dioxane (3 mL) was heated under reflux for 1 h. After completion of the reaction (monitored by DC, HPLC) the reaction mixture was cooled to room temperature, diluted with water (10 mL) and extracted with ethyl acetate (3x10 mL). The organic phase was dried (Na₂SO₄), filtered and evaporated to afford a crude product. Chromatography affords the title compound as beige-yellow solid (66-88%).

The compounds listed in table I can be prepared in an analogous manner.

**Table I**

| | | | | |
|---|---|---|---|---|
| 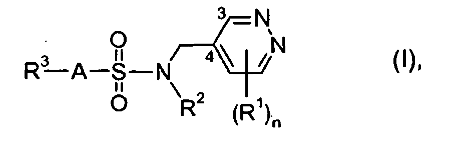 | | | | |

| ex. no | (R¹)ₙ | R² | A-R³ | m.p. [°C]/ |
|---|---|---|---|---|
| | | | | RT [min] |
| 1. | - | H | 4-bromo-thiophen-2-yl | 120-122 |
| 2. | - | H | 4-(4-fluorophenyl)-thiophen-2-yl | 123 |
| 3. | - | H | 4-(3,4-dichlorophenyl)-phenyl | 3.15 |
| 4. | - | H | 4-(3-trifluoromethylphenyl)-phenyl | 3.02 |
| 5. | - | H | 4-(4-trifluoromethylphenyl)-phenyl | 3.05 |
| 6. | - | H | 4-(3-chlorophenyl)-phenyl | 2.91 |
| 7. | - | H | 4-(2,4-difluorophenyl)-phenyl | 2.74 |
| 8. | - | H | 4-(3,4-difluorophenyl)-phenyl | 2.79 |
| 9. | - | H | 4-(3-CN-phenyl)-phenyl | 2.48 |
| 10. | - | H | 4-(phenyl)-phenyl | 2.63 |
| 11. | - | H | 4-(4-CN-phenyl)-phenyl | 2.49 |
| 12. | - | H | 4-(2-trifluoromethylphenyl)-phenyl | 2.87 |
| 13. | - | H | 4-(2,4-dichlorophenyl)-phenyl | 3.12 |
| 14. | - | H | 4-(2-fluorophenyl)-phenyl | 2.67 |
| 15. | - | H | 4-(3-fluorophenyl)-phenyl | 2.71 |
| 16. | - | H | 4-(4-methoxyphenyl)-phenyl | 2.65 |
| 17. | - | H | 4-(4-fluorophenyl)-phenyl | 2.70 |
| 18. | - | H | 4-(4-chlorophenyl)-phenyl | 2.94 |
| 19. | - | H | 4-(3-methoxyphenyl)-phenyl | 2.68 |
| 20. | - | H | 4-(3-methylphenyl)-phenyl | 104 |
| 21. | - | H | 5-(2,4-dichlorophenyl)-thiophen-2-yl | 114-116 |
| 22. | - | H | 5-(3,4difluorophenyl)-thiophen-2-yl | 129 |
| 23. | - | H | 5-(3-cyanophenyl)-thiophen-2-yl | 163-166 |
| 24. | - | H | 6-(2-fluorophenyl)-pyridin-3-yl | 98-106 |
| 25. | - | H | 4-(2-methoxyphenyl)-phenyl | 66-139 |
| 26. | - | H | 4-(2-methylphenyl)-phenyl | 79-83 |
| 27. | - | H | 6-(phenyl)-pyridin-3-yl | 157 |
| 28. | - | H | 6-(2-chlorophenyl)-pyridin-3-yl | 130 |
| 29. | - | H | 6-(3-chlorophenyl)-pyridin-3-yl | 127 |
| 30. | - | H | 5-(3-CF₃-phenyl)-thiophen-2-yl | 90 |
| 31. | - | H | 5-(2-CF₃-phenyl)-thiophen-2-yl | 109-112 |
| 32. | - | H | 5-(phenyl)-thiophen-2-yl | 100-112 |
| 33. | - | H | 5-(3-fluoro-phenyl)-thiophen-2-yl | 120 |
| 34. | - | H | 4-(4-methylphenyl)-phenyl | 178-182 |
| 35. | - | H | 5-(2-methoxy-phenyl)-thiophen-2-yl | 144-149 |
| 36. | - | H | 4-(2-chlorophenyl)-phenyl | 92 |
| 37. | - | H | 5-(3-methoxy-phenyl)-thiophen-2-yl | 122 |
| 38. | - | H | 5-(2,4-difluoro-phenyl)-thiophen-2-yl | 154-160 |
| 39. | - | H | 5-(4-chloro-phenyl)-thiophen-2-yl | 120 |
| 40. | - | H | 5-(3-chloro-phenyl)-thiophen-2-yl | 2.93 |
| 41. | - | H | 5-(2-chloro-phenyl)-thiophen-2-yl | 2.73 |
| 42. | - | H | 5-(4-fluoro-phenyl)-thiophen-2-yl | 146 |
| 43. | - | H | 5-(2-fluoro-phenyl)-thiophen-2-yl | 105 |
| 44. | - | H | 5-(2-methyl-phenyl)-thiophen-2-yl | 81 |
| 45. | - | H | 5-(4-methoxy-phenyl)-thiophen-2-yl | 151-154 |
| 46. | - | H | 5-(4-CF₃-phenyl)-thiophen-2-yl | 90 |
| 47. | - | H | 6-(3-methylphenyl)-pyridin-3-yl | 125 |
| 48. | - | H | 6-(4-methylphenyl)-pyridin-3-yl | 192 |
| 49. | - | H | 6-(4-methoxyphenyl)-pyridin-3-yl | 173 |
| 50. | - | H | 6-(2-CF₃-phenyl)-pyridin-3-yl | 152 |
| 51. | - | H | 6-(3-CF₃-phenyl)-pyridin-3-yl | 96 |
| 52. | - | H | 6-(4-cyano-phenyl)-pyridin-3-yl | 189 |
| 53. | - | H | 6-(2,4-difluoro-phenyl)-pyridin-3-yl | 167 |
| 54. | - | H | 6-(3,4-difluoro-phenyl)-pyridin-3-yl | 160 |
| 55. | - | H | 6-(2,4-dichloro-phenyl)-pyridin-3-yl | 150 |
| 56. | - | H | 4-(3-fluoro-phenyl)-thiophen-2-yl | 2.60 |
| 57. | - | H | 5-(4-cyano-phenyl)-thiophen-2-yl | 170 |
| 58. | - | H | 5-(4-methyl-phenyl)-thiophen-2-yl | 144 |
| 59. | - | H | 5-(3-methyl-phenyl)-thiophen-2-yl | 2.87 |
| 60. | - | H | 6-(4-chlorophenyl)-pyridin-3-yl | 178 |
| 61. | - | H | 6-(3-fluorophenyl)-pyridin-3-yl | 145 |
| 62. | - | H | 6-(4-fluorophenyl)-pyridin-3-yl | 165 |
| 63. | - | H | 6-(2-methylphenyl)-pyridin-3-yl | 86 |
| 64. | - | H | 6-(2-methoxyphenyl)-pyridin-3-yl | 170 |
| 65. | - | H | 6-(3-methoxyphenyl)-pyridin-3-yl | 134 |
| 66. | - | H | 6-(4-CF₃-phenyl)-pyridin-3-yl | 196 |
| 67. | - | H | 4-(phenyl)-thiophen-2-yl | 135 |
| 68. | - | H | 4-(4-chlorophenyl)-thiophen-2-yl | 105 |
| 69. | - | H | 4-(2-chlorophenyl)-thiophen-2-yl | 50 |
| 70. | - | H | 4-(4-fluorophenyl)-thiophen-2-yl | 123 |
| 71. | - | H | 4-(2-fluorophenyl)-thiophen-2-yl | 83 |
| 72. | - | H | 4-(4-methylphenyl)-thiophen-2-yl | 2.82 |
| 73. | - | H | 4-(3-methylphenyl)-thiophen-2-yl | 105 |
| 74. | - | H | 4-(2-methylphenyl)-thiophen-2-yl | 86 |
| 75. | - | H | 4-(4-methoxyphenyl)-thiophen-2-yl | 105 |
| 76. | - | H | 4-(3-methoxyphenyl)-thiophen-2-yl | 133 |
| 77. | - | H | 4-(3-CF₃-phenyl)-thiophen-2-yl | 143 |
| 78. | - | H | 4-(3-CN-phenyl)-thiophen-2-yl | 181 |
| 79. | - | H | 4-(3,4-difluoro-phenyl)-thiophen-2-yl | 138 |
| 80. | - | H | 4-(3,4-dichloro-phenyl)-thiophen-2-yl | 158 |
| 81. | - | H | 6-(3,4-dichlorophenyl)-pyidin-3-yl | 158 |
| 82. | - | H | 4-(2,4-difluoro-phenyl)-thiophen-2-yl | 141 |
| 83. | - | H | 4-(2,4-dichloro-phenyl)-thiophen-2-yl | 157 |
| 84. | - | H | 6-(3-CN-phenyl)-pyridin-3-yl | 155 |
| 85. | - | H | 4-(3-chloro-phenyl)-thiophen-2-yl | 121 |
| 86. | - | H | 4-(2-methoxy-phenyl)-thiophen-2-yl | 112 |
| 87. | - | H | 4-(4-CF₃-phenyl)-thiophen-2-yl | 118 |
| 88. | - | H | 4-(2-CF₃-phenyl)-thiophen-2-yl | 2.78 |
| 89. | - | H | 4-(4-CN-phenyl)-thiophen-2-yl | 180 |
| 90. | - | H | 4-(4-Cl-phenoxy)-phenyl | 176 |
| 91. | - | H | 4-(2,4-difluorophenoxy)-phenyl | 88 |

| | | | | |
|---|---|---|---|---|
| - when referring to (R¹)ₙ means that n is zero. m.p. melting point RT retention time, determined by HPLC; HPLC column: RP-18 column (Chromolith Speed ROD from Merck KgaA, Germany). Elution: acetonitrile + 0.1 % trifluoroacetic acid (TFA) / water in a ratio of from 5:95 to 95:5 in 5 minutes at 40°C. | | | | |

### Examples of the action against harmful fungi

The fungicidal action of the compounds of the formula I was demonstrated by the following experiments:

### Greenhouse tests:

The active compounds were formulated separately or together as a stock solution comprising 25 mg of active compound which was made up to 10 ml using a mixture of acetone and/or dimethyl sulfoxide (DMSO) and the emulsifier Wettol (wetting agent having emulsifying and dispersing action based on ethoxylated alkylphenols) in a volume ratio of solvent/emulsifier of 99 to 1. This solution was then made up to 100 ml using water. This stock solution was diluted with the solvent/emulsifier/water mixture described to the active compound concentration given below.

### Use example 1 - Activity against early blight on tomatoes caused by Phytophthora infestans with protective application

Young seedlings of tomato plants were grown in pots. The plants were sprayed to runoff with an aqueous suspension containing the concentration of active ingredient stated below. The next day, the treated plants were inoculated with an aqueous suspension of sporangia of Phytophthora infestans. After inoculation, the trial plants were immediately transferred to a humid chamber. After six days at 18 to 20°C and a relative humidity close to 100%, the extent of fungal attack on the leaves was visually assessed as % diseased leaf area.

In this test, the plants which had been treated with 250 ppm of the active compound from examples 3, 67, 70, 71 and 89, respectively, showed an infection of less than or equal to 10% whereas the untreated plants were 90% infected.

### Use example 2 - Protective action against Puccinia recondita on wheat (brown rust of wheat)

Leaves of potted wheat seedlings of the cultivar "Kanzler" were sprayed to runoff point with an aqueous suspension having the concentration of active compound stated below. The next day, the treated plants were dusted with a suspension of spores of brown rust of wheat (Puccinia recondita). The plants were then placed in a chamber with high atmospheric humidity (90 to 95%), at 20-22°C, for 24 hours. During this time, the spores germinated and the germinal tubes penetrated into the leaf tissue. The next day, the test plants were returned into the greenhouse and cultivated at temperatures between 20 and 22°C and at 65 to 70% relative atmospheric humidity for a further 7 days. The extent of the rust development on the leaves was then determined visually.

### Use example 3 - Activity against soyabean rust caused by Phakospora pachyrhizi

Leaves of potted soyabean seedlings of the cultivar "Oxford" were inoculated with a spore suspension of soyabean rust (Phakospora pachyrhizi). The pots were then placed in a chamber at high atmospheric humidity (90 to 95%) and 23 to 27°C for 24 hours. During this time, the spores germinated and the germ tubes penetrated into the leaf tissue. The next day, the infected plants were sprayed to runoff point with an aqueous solution having the active compound concentration stated below. The solution had been prepared as described above. After the spray coating had dried, the test plants were cultivated in a greenhouse at temperatures between 23 and 27°C and at 60 to 80% relative atmospheric humidity for 14 days. The extent of the rust fungus development on the leaves was then determined.

### Microtiter tests

The active compounds were formulated separately as a stock solution in dimethyl sulfoxide (DMSO) at a concentration of 10 000 ppm.

### Use example 4 - Activity against the late blight pathogen Phytophthora infestans in the microtiter test

The stock solution was pipetted into a microtiter plate (MTP) and diluted to the stated active compound concentration using a pea juice-based aqueous nutrient medium for fungi. An aqueous zoospore suspension of Phytophthora infestans was then added. The plates were placed in a water vapor-saturated chamber at temperatures of 18°C. Using an absorption photometer, the MTPs were measured at 405 nm on day 7 after the inoculation. The measured parameters were compared to the growth of the active compound-free control variant (= 100%) and the fungus- and active compound-free blank value to determine the relative growth in % of the pathogens in the individual active compounds.

In this test, the sample which had been treated with 125 ppm of the active compound from examples 4, 5, 6, 7, 8, 10, 13, 14, 15, 17, 19, 20, 22, 24, 25, 26, 30, 32, 33, 34, 36, 42, 43, 44, 51, 68, 69, 75, 83, 86, and 88, respecitvely showed up to at most 25% growth of the pathogen.

### Use example 5 - Activity against the rice blast pathogen caused by Pyricularia oryzae in the microtiter test

The stock solution was pipetted into a microtiter plate (MTP) and diluted to the stated active compound concentration using a malt-based aqueous nutrient medium for fungi. An aqueous spore suspension of Pyricularia oryzae was then added. The plates were placed in a water vapor-saturated chamber at temperatures of 18°C. Using an absorption photometer, the microtiter plates were measured at 405 nm on day 7 after the inoculation. The measured parameters were compared to the growth of the active compound-free control variant (= 100%) and the fungus- and active compound-free blank value to determine the relative growth in % of the pathogens in the individual active compounds.

In this test, the sample which had been treated with 125 ppm of the active compound from examples 4, 7, 8, 13, 17, 20, 30, 31, 37, 40, 44, 66, 83, 87, 90 and 91, respectively, showed up to at most 25% relative growth of the pathogen

### Use example 6 - Activity against the Septoria blotch pathogen caused by Septoria tritici in the microtiter test

The stock solution was pipetted into a microtiter plate (MTP) and diluted to the stated active compound concentration using a malt-based aqueous nutrient medium for fungi. An aqueous spore suspension of Septoria tritici was then added. The plates were placed in a water vapor-saturated chamber at temperatures of 18°C. Using an absorption photometer, the microtiter plates were measured at 405 nm on day 7 after the inoculation. The measured parameters were compared to the growth of the active compound-free control variant (= 100%) and the fungus- and active compound-free blank value to determine the relative growth in % of the pathogens in the individual active compounds. In this test, the sample which had been treated with 125 ppm of the active compound from examples 39, 40, 46, 59, 72, 74, 90 and 91, respectively, showed up to at most 25% relative growth of the pathogen.

The action of the compounds of the formula I against harmful pests was demonstrated by the following experiments:

### Use example 7 - Activity against Boll weevil (Anthonomus grandis)

The active compounds were formulated in 1:3 dimethylsulfoxide / water. 10 to 15 eggs were placed into microtiterplates filled with 2% agar-agar in water and 300 ppm formaline. The eggs were sprayed with 20 µl of the test solution, the plates were sealed with pierced foils and kept at 24-26°C and 75-85% humidity with a day/night cycle for 3 to 5 days. Mortality was assessed on the basis of the remaining unhatched eggs or larvae on the agar surface and/or quantity and depth of the digging channels caused by the hatched larvae. Tests were replicated 2 times.
In this test the eggs which have been treated with 2500 ppm of the active compounds of examples 5 and 11, respecitvely, showed a mortality of at least 75%.

### Use example 8 - Activity against Mediterranean fruittly (Ceratitis capitata)

The active compounds were formulated in 1:3 dimethylsulfoxide / water. 50 to 80 eggs were placed into microtiterplates filled with 0.5% agar-agar and 14 % diet in water. The eggs were sprayed with 5 µl of the test solution, the plates were sealed with pierced foils and kept at 27-29°C and 75-85% humidity under fluorescent light for 6 days. Mortality was assessed on the basis of the agility of the hatched larvae. Tests were replicated 2 times.

In this test the eggs which have been treated with 2500 ppm of the active compound of example 60 showed a mortality of at least 75%.

### Use example 9 - Activity against Tobacco budworm (Heliothis virescens)

The active compounds were formulated in 1:3 dimethylsulfoxide / water. 15 to 25 eggs were placed into microtiterplates filled with diet. The eggs were sprayed with 10 µl of the test solution, the plates were sealed with pierced foils and kept at 27-29°C and 75-85% humidity under fluorescent light for 6 days. Mortality was assessed on the basis of the agility and of comparative feeding of the hatched larvae. Tests were replicated 2 times. In this test the eggs which have been treated with 2500 ppm of the active compound from example 11 showed a mortality of at least 75%.

### Use example 10 - Activity against Vetch aphid (Megoura viciae)

The active compounds were formulated in 1:3 dimethylsulfoxide / water. Bean leaf disks were placed into microtiterplates filled with 0.8% agar-agar and 2.5 ppm OPUS^{®}. The leaf disks were sprayed with 2.5 µl of the test solution and 5 to 8 adult aphids were placed into the microtiterplates which were then closed and kept at 22-24°C and 35-45% under fluorescent light for 6 days. Mortality was assessed on the basis of vital, reproduced aphids. Tests were replicated 2 times.

## Claims

1. Pyridazin-4-ylmethyl-sulfonamide compounds of the formula (I) 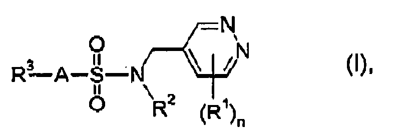 where:
n Is zero, one, two or three;
R¹ is halogen, C₁-C₄-alkyl, C₁-C₄-haloalkyl, C₁-C₄-alkoxy, C₁-C₄-haloalkoxy, C₁- C₄-alkylthio, C₁-C₄-haloalkylthio, C₁-C₄-alkylsulfinyl, C₁-C₄-haloalkylsulfinyl, C₁-C₄-alkylsulfonyl, C₁-C₄-haloalkylsulfonyl, amino, C₁-C₄-alkylamino, di(C₁- C₄-alkyl)amino, C₁-C₄-alkoxy-C₁-C₄-alkyl, C₁-C₄-haloalkoxy-C₁-C₄-alkyl, C₂- C₄-alkenyl, C₂-C₄-alkynyl, C₃-C₈-cycloalkyl or C₁-C₄-alkyl-C₃-C₈-cycloalkyl; and/or one radical R¹ that is bound to a carbon atom adjacent to a nitrogen atom of the pyridazine ring may form together with said carbon atom and nitrogen atom a fused five-membered aromatic heterocycle, which may contain one, two or three further nitrogen atoms as ring members; or two radicals R¹ that are bound to adjacent carbon atoms of the pyridazine ring may form together with said carbon atoms a fused benzene ring, a fused saturated or partially unsaturated 5-, 6-, or 7-membered carbocycle or a fused 5-, 6-, or 7-membered heterocycle containing one, two or three heteroatoms selected from the group consisting of 2 nitrogen, 1 oxygen and 1 sulfur atoms as ring members, it being possible for the fused ring to carry one or two radicals selected from the group consisting of halogen, C₁-C₄- alkyl, halomethyl, C₁-C₄-alkoxy or halomethoxy;
R² is hydrogen, C₁-C₄-alkyl, C₁-C₄-haloalkyl, C₁-C₄-alkoxy, C₁-C₄-haloalkoxy, C₁-C₄-alkylamino, di(C₁-C₄-alkyl)amino, C₁-C₄-alkoxy-C₁-C₄-alkyl, C₁-C₄- haloalkoxy-C₁-C₄-alkyl, C₂-C₄-alkenyl, C₂-C₄-haloalkenyl, C₂-C₄-alkynyl, C₃- C₈-cycloalkyl, C₁-C₄-alkyl-C₃-C₈-cycloalkyl or benzyl wherein the phenyl moiety of benzyl is unsubstituted or carries 1, 2 , 3, 4, or 5 substituents selected from the group consisting of cyano, halogen, C₁-C₄-alkyl, C₁-C₄-
A haloalkyl, C₁-C₄-alkoxy, C₁-C₄-haloalkoxy, (C₁-C₄-alkoxy)carbonyl and di(C₁-C₄-alkyl)aminocarbonyl; is phenylene, 5- or 6-membered heteroarenediyl, where heteroarenediyl contains one, two, three or four heteroatoms selected from the group consisting of 1, 2, 3 or 4 nitrogen atoms, 1 oxygen atom and 1 sulfur atom, as ring members, and where phenylene and heteroarenediyl for their part are unsubstituted or carry 1, 2, 3 or 4 substituents R^{A}, each selected from the group consisting of cyano, halogen, C₁-C₄-alkyl, C₁-C₄-haloalkyl, C₁-C₄- alkoxy, C₁-C₄-haloalkoxy, C₂-C₄-alkenyl, C₂-C₄-haloalkenyl, C₂-C₄-alkynyl, C₂-C₄-haloalkynyl, (C₁-C₄-alkoxy)carbonyl and di(C₁-C₄- alkyl)aminocarbonyl, or
A is C₁-C₈-alkanediyl, C₁-C₈-haloalkanediyl, C₂-C₈-alkenediyl, C₂-C₈- haloalkenediyl, C₂-C₈-alkynediyl, or C₂-C₈-haloalkynediyl, wherein the six last-mentioned radicals are unsubstituted or carry one, two or three substituents R^{AA}, each selected from the group consisting of cyano, C₁-C₄- alkoxy, C₁-C₄-haloalkoxy, (C₁-C₄-alkoxy)carbonyl and di(C₁-C₄- alkyl)aminocarbonyl;
R³ is hydrogen, C₁-C₄-alkoxy, C₁-C₄-haloalkoxy, C₁-C₄-alkylthio, C₁-C₄- haloalkylthio,C₁-C₄-alkylamino, di(C₁-C₄-alkyl)amino, (C₁-C₄-alkyl)carbonyl, (C₁-C₄-alkoxy)iminomethyl, acryloyl (vinylcarbonyl), C₃-C₈-cycloalkyl, C₁-C₄- alkyl-C₃-C₈-cycloalkyl, or C₅-C₈-cycloalkenyl, phenyl, benzyl, phenoxy, phenylthio, a 5- or 6-membered heteroaryl radical, wherein the heteroaryl ring has 1, 2, 3 or 4 heteroatoms selected from the group consisting of 1, 2, 3 or 4 nitrogen atoms, 1 oxygen atom and 1 sulfur atom, as ring members, it being possible for the heteroaryl ring and the phenyl ring of phenyl, benzyl, phenoxy and phenylthio to be unsubstituted or substituted by one, two or three substituents R^{B}, each selected from the group consisting of cyano, nitro, amino, halogen, C₁-C₄-alkyl, C₁-C₄- haloalkyl, C₁-C₄-alkoxy, C₁-C₄-haloalkoxy, (C₁-C₄-alkoxy)carbonyl, di(C₁-C₄- alkyl)aminocarbonyl, C₁-C₄-alkylthio, C₁-C₄-alkylsulfinyl and C₁-C₄- alkylsulfonyl; or a 5- or 6-membered heteroaryloxy radical or a 5- or 6-membered heteroarylthio radical, wherein the heteroaryl ring in the two aforementioned radicals has 1, 2, 3 or 4 heteroatoms selected from the group consisting of 1, 2, 3, or 4 nitrogen atoms, 1 oxygen atom and 1 sulfur atom, as ring members, it being possible for said heteroaromatic ring to carry one, two or three substituents R^{B}; or if A is phenylene or 5- or 6-membered heteroarenediyl, the radical R³ together with a radical R^{A} may form together with the carbon atoms to which they are bound a fused benzene ring, wherein the fused benzene ring may be unsubstituted or may carry 1, 2 or 3 substituents selected, independently from one another, from the group consisting of halogen, cyano, nitro, C₁-C₄-alkyl, C₁-C₄-haloalkyl, C₁-C₄-alkoxy, C₁-C₄-haloalkoxy, (C₁-C₄-alkoxy)carbonyl and di(C₁-C₄-alkyl)aminocarbonyl, or if A is phenylene or 5- or 6-membered heteroarenediyl, the radical R³ can also be C₁-C₄-alkyl, C₂-C₄-alkenyl or C₂-C₄-alkyrlyl;
and the N-oxides, the agriculturally acceptable salts and the veterinarily acceptable salts of the compounds of the formula (I), except for the compound N-(3,6-dichloro-pyridazin-4ylmethyl)-4-methylbenzenesulfonamide.

2. Compounds of the formula (I) as claimed in claim 1, in which A is phenylene or heteroarenediyl, which both may be unsubstituted or may carry one, two or three substituents R^{A}.

3. Compounds of the formula (I) as claimed in claim 2, in which A is 1,3- or 1,4-phenylene, which may carry one, two or three substituents R^{A}.

4. Compounds of the formula (I) as claimed in claim 2, in which A is heteroarenediyl, selected from the group consisting of thiophene-2,5-diyl, thiophene-2,4-diyl, thiophene-3,5-diyl, thiazole-2,5-diyl, thiazole-2,4-diyl, oxazole-2,5-diyl, oxazole-2,4-diyl, pyrazole-3,5-diyl, pyrazole-1,3-diyl, pyrazole-1,4-diyl, pyridine-2,5-diyl. pyridine-2,6-diyl, pyridine-2,4-diyl, pyridine-3,8-diyl, wherein heteroarenediyl is unsubstituted or carries one, two or three substituents R^{A}.

5. Compounds of the formula (I) according to claim 1, in which R³ is phenyl or phenoxy, where the two last-mentioned radicals are unsubstituted or carry one, two, or three substituents R^{B}.

6. Compounds of the formula (I) as claimed in claim 1, in which R² is hydrogen.

7. Compounds of the formula (I) as claimed in any of the preceding claims, in which n is zero.

8. A process for preparing pyridazin-4-ylmethyl-sulfonamide compounds of the formula (I) as defined in claim 1, which comprises reacting an aminomethylpyridazine compound of the formula (II) 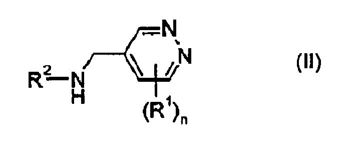 in which n, R¹ and R² are as defined in claim 1,
under basic conditions with a sulfonic acid derivative of the formula (III) 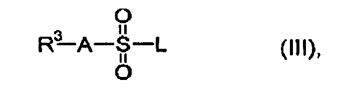 in which A and R³ are as defined in claim 1 and L is hydroxy or halogen.

9. An agricultural composition which comprises a solid or liquid carrier and at least one compound of the formula (I) or an N-oxide or an agriculturally acceptable salt thereof, according to claim 1.

10. A method for the treatment of phytopathogenic harmful fungi, which process comprises treating the fungi or the materials, plants, the soil or seeds to be protected against fungal attack, with an effective amount of at least one compound of the formula (I) or an or an N-oxide or an agriculturally acceptable salt thereof, according to claim 1.

11. The use of compounds of the formula (I), their N-oxides and their agriculturally acceptable salts, according to claim 1, and of compositions comprising compounds of formula I, for combating phytopathogenic harmful fungi.

12. The use of compounds of the formula (I) and the N-oxides and the agriculturally acceptable salts, according to claim 1, and of compositions comprising at least one compound of formula (I), for protecting seed, the seedlings' roots and shoots from infestation by phytopathogenic harmful fungi and/or arthropod pests.

13. Seed comprising a compound of the formula (I), or an N-oxide or an agriculturally acceptable salt thereof, as defined in claim 1, in an amount of from 0.1 g to 10 kg per 100 kg of seed.

## Patentansprüche

1. Pyridazin-4-ylmethylsulfonsäureamidverbindungen der Formel (I) 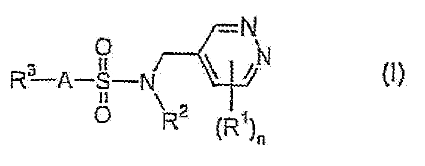 wobei:
n für null, eins, zwei oder drei steht;
R¹ für Halogen, C₁-C₄-Alkyl, C₁-C₄-Halogenalkyl, C₁-C₄-Alkoxy, C₁-C₄-Halogenalkoxy, C₁-C₄- Alkylthio, C₁-C₄-Halogenalkylthio, C₁-C₄- Alkylsulfonyl, C₁-C₄-Halogenalkylsulfinyl, C₁- C₄-Alkylsulfonyl, C₁-C₄-Halogenalkylsulfonyl, Amino, C₁-C₄-Alkylamino, Di(C₁-C₄-alkyl)amino, C₁-C₄-Alkoxy-C₁-C₄-alkyl, C₁-C₄-Halogenalkoxy-C₁- C₄-alkyl, C₂-C₄-Alkenyl, C₂-C₄-Alkinyl, C₃-C₈- Cycloalkyl oder C₁-C₄-Alkyl-C₃-C₈-cycloalkyl steht; und/oder ein an einem einem Stickstoffatom des Pyridazinrings benachbarten Kohlenstoffatom gebundener Rest R¹ zusammen mit diesem
R² Kohlenstoffatom und diesem Stickstoffatom einen kondensierten fünfgliedrigen aromatischen Heterocyclus bilden kann, der ein, zwei oder drei weitere Stickstoffatome als Ringglieder enthalten kann; oder zwei an benachbarte Kohlenstoffatome des Pyridazinrings gebundene Reste R¹ zusammen mit diesen Kohlenstoffatomen einen kondensierten Benzolring, einen kondensierten gesättigten oder teilweise ungesättigen 5-, 6- oder 7- gliedrigen Carbocyclus oder einen kondensierten 5-, 6- oder 7-gliedrigen Heterocyclus mit einem, zwei oder drei Heteroatomen ausgewählt aus der aus 2 Stickstoff- 1 Sauerstoff- und 1 Schwefelatomen bestehenden Gruppe als Ringgliedern, bilden können, wobei es möglich ist, dass der kondensierte Ring einen oder zwei Reste ausgewählt aus der aus Halogen, C₁- C₄-Alkyl, Halogenmethyl, C₁-C₄-Alkoxy und Halogenmethoxy bestehenden Gruppe tragen kann; für Wasserstoff, C₁-C₄-Alkyl, C₁-C₄- Halogenalkyl, C₁-C₄-Alkoxy, C₁-C₄-Halogenallcoxy, C₁-C₄-Alkylamino, Di(C₁-C₄-alkyl)amino, C₁-C₄- Alkoxy-C₁-C₄-alkyl, C₁-C₄-Halogenalkoxy-C₁-C₄- alkyl, C₂-C₄-Alkenyl, C₂-C₄-Halogenalkenyl, C₂- C₄-Alkinyl, C₃C₈-Cycloalkyl, C₁-C₄-Alkyl-C₃-C₈- cycloalkyl oder Benzyl steht, wobei die Phenyleinheit des Benzyls unsubstituiert ist oder 1, 2, 3, 4 oder 5 Substituenten ausgewählt aus der aus Cyano, Halogen, C₁-C₄- Alkyl, C₁-C₄-Halogenalkyl, C₁-C₄-Alkoxyl, C₁-C₄- Halogenalkoxy, (C₁-C₄-Alkoxy)carbonyl und Di(C₁-C₄-alkyl) aminocarbonyl bestehenden Gruppe trägt;
A für Phenylen oder 5- oder 6-gliedriges Heteroarendiyl steht, wobei Heteroarendiyl eines, zwei, drei oder vier Heteroatome ausgewählt aus der aus 1, 2, 3 oder 4 Stickstoffatomen, 1 Sauerstoffatom und 1 Schwefelatom bestehenden Gruppe als Ringglieder enthält, und wobei Phenylen und Heteroarendiyl ihrerseits unsubstituiert sind oder 1, 2, 3 oder 4 Substituenten R^{A} tragen, die jeweils aus der aus Cyano, Halogen, C₁-C₄- Alkyl, C₁-C₄-Halogenalkyl, C₁-C₄-Alkoxy, C₁-C₄- Halogenalkoxy, C₂-C₄-Alkenyl, C₂-C₄- Halogenalkenyl, C₂-C₄-Alkinyl, C2-C₄- Halogenalkinyl, (C₁-C₄-Alkoxy)carbonyl und Di(C₁-C₄-alkyl)aminocarbonyl bestehenden Gruppe ausgewählt sind, oder
A für C₁-C₈-Alkandiyl, C₁-C₈-Halogenalkandiyl, C₂- C₈-Alkendiyl, C₂-C₈-Halogenalkendiyl, C₂-C₈- Alkindiyl oder C₂-C₈-Halogenalkindiyi steht, wobei die sechs letztgenannten Reste unsubstituiert sind oder einen, zwei oder drei Substituenten R^{AA}, jeweils ausgewählt aus der aus Cyano, C₁-C₄-Alkoxy, C₁-C₄-Halogenalkoxy, (C₁-C₄-Alkoxy)carbonyl und Di(C₁-C₄- alkyl)aminocarbonyl bestehenden Gruppe, tagen;
R³ für Wasserstoff, C₁-C₄-Alkoxy, C₁-C₄- Halogenalkoxy, C₁-C₄-Alkylthio, C₁-C₄- Halogenalkylthio, C₁-C₄-Alkylamino, Di (C₁-C₄- alkyl)amino, (C₁-C₄-Alkyl)carbonyl, (C₁-C₄- Alkoxy)iminomethyl, Acryloyl (Vinylcarbonyl), C₃-C₈-Cycloalkyl, C₁-C₄-Alkyl-C₃-C₈-cycloalkyl oder C₅-C₈-Cycloalkenyl, Phenyl, Benzyl, Phenoxy, Phenylthio, einen 5- oder 6-gliedrigen Heteroarylrest, wobei der Heteroarylring 1, 2, 3 oder 4 Heteroatome ausgewählt aus der aus 1, 2, 3 oder 4 Stickstoffatomen, 1 Sauerstoffatom und 1 Schwefelatom bestehenden Gruppe als Ringglieder aufweist, wobei der Heteroarylring und der Phenylring von Phenyl, Benzyl, Phenoxy und Phenylthio unsubstituiert oder durch einen, zwei oder drei Substituenten R^{B} jeweils ausgewählt aus der aus Cyano, Nitro, Amino, Halogen, C₁-C₄-Alkyl, C₁-C₄-Halogenalkyl, C₁-C₄- Alkoxy, C₁-C₄-Halogenalkoxy, (C₁-C₄- Alkoxy) carbonyl, Di(C₁-C₄-alkyl)aminocarbonyl, C₁-C₄-Alkylthio, C₁-C₄ -Alkylsulfinyl und C₁-C₄- Alkylsulfonyl bestehenden Gruppe substituiert seien können; oder einen 5- oder 6-gliedrigen Heteroaryloxyrest oder einen 5- oder 6-gliedrigen Heteroarylthiorest steht, wobei der Heteroarylring in den beiden oben erwähnter Resten 1, 2, 3 oder 4 Heteroatome ausgewählt aus der aus 1, 2, 3 oder 4 Stickstoffatomen, 1 Sauerstoffatom und 1 Schwefelatom bestehenden Gruppe als Ringglieder aufweist, wobei der heteroaromatische Ring eines, zwei oder drei Substituenten R³ tragen kann; oder, wenn A für Phenylen oder 5- oder 6- gliedriges Heteroarendiyl steht, der Rest R³ zusammen mit einem Rest R^{A} zusammen mit den Kohlenstoffatomen, an die sie gebunden sind, einen kondensierten Benzolring bilden kann, wobei der kondensierte Benzolring unsubstituiert sein kann oder eins, zwei oder drei Substituenten unabhängig voneinander ausgewählt aus der aus Halogen, Cyano, Nitro, C₁-C₄-Alkyl, C₁-C₄-Halogenalkyl, C₁-C₄-Alkoxy C₁-C₄-Halogenalkoxy, (C₁-C₄-Alkoxy) carbonyl und Di (C₁-C₄-alkyl) aminocarbonyl bestehenden Gruppe tragen kann, oder wenn A für Phenylen oder 5- oder 6-gliedriges Heteroarendiyl steht, der Rest R³ auch für C₁- C₄-Alkyl, C₂-C₄-Alkenyl oder C₂-C₄-Alkinyl stehen kann;
und die N-Oxide, die landwirtschaftlich unbedenklichen Salze und die veterinärmedizinisch unbedenklichen Salze der Verbindungen der Formel (I) mit Ausnahme der Verbindung N-(3,6-Dichlorpyridazin-4-ylmethyl)-4-mehtylbenzolsulfonsäureamid.

2. Verbindungen der Formel (I) nach Anspruch 1, in denen A für Phenylen oder Heteroarendiyl steht, die beide unsubstituiert sein können oder einen, zwei oder drei Substituenten R^{A} tragen können.

3. Verbindungen der Formel (I) nach Anspruch 2, in denen A für 1,3- oder 1,4-Phenylen steht, das eignen, zwei oder drei Substituenten R^{A} tragen kann.

4. Verbindungen der Formel (I) nach Anspruch 2, in denen A für Heteroarendiyl ausgewählt aus der aus Thiophen-2,5-diyl, Thiophen-2,4-diyl, Thiophen-3,5-diyl, Thiazol-2,5-diyl, Thiazol-2,4-diyl, Oxazol-2,5-diyl, Oxazol-2,4-diyl, Pyrazol-3,5-diyl, Pyrazol-1,3-diyl, Pyrazol-1,4-diyl, Pyridin-2,5-diyl, Pyridin-2,6-diyl, Pyridin-2,4-diyl und Pyridin-3,5-diyl bestehenden Gruppe steht, wobei Heteroarendiyl unsubstituiert ist oder einen, zwei oder drei Substituenten R^{A} trägt.

5. Verbindungen der Formel (I) nach Anspruch 1, in denen R³ für Phenyl oder Phenoxy steht, wobei die beiden letztgenannten Reste unsubstituiert sind oder einen, zwei oder drei Substituenten R³ tragen.

6. Verbindungen der Formel (I) nach Anspruch 1, in denen R² für Wasserstoff steht.

7. Verbindungen der Formel (I) nach einem der vorhergehenden Ansprüche, in denen n für null steht.

8. Verfahren zur Herstellung von pyridazin-4-ylmethylsulfonsäureamidverbindungen der Formel (I) nach Anspruch 1, bei dem man eine Aminomethylpyridazinverbindung der Formel (II) 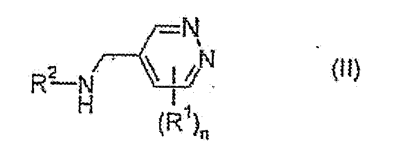 in welcher n, R¹ und R² wie in Anspruch 1 definiert sind,
unter basischen Bedingungen mit einem Sulfonsäurederivat der Formel (III) 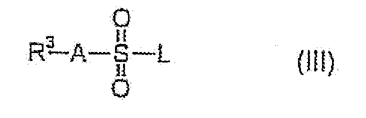 in welcher A und R³ wie in anspruch 1 definiert sind und L für Hydroxy oder Halogen steht, umsetzt.

9. Landwirtschaftliche Zusammensetzung, enthaltend eignen festen oder flüssigen Träger und wenigstens eine Verbindung der Formel (I) oder ein N-Oxid oder ein landwirtschaftlich unbedenkliches Salz davon nach Aspruch 1.

10. Verfahren zur Behandlung von phytopathogenen Schadpilzen, bei dem man die Pilze oder die gegen Pilzbefall zu schützenden Materialien, Pflanzen, den Boden oder Samen mit einer wirksamen Menge wenigstens einer Verbindung der Formel (I) oder eines N-Oxids oder eines landwirtschaftlich unbedenklichen Salzes davon nach Anspruch 1 behandelt.

11. Verwendung von verbindungen der Formel (I), deren N-Oxiden und deren landwirtschaftlich unbedenklichen Salzen nach Anspruch 1 und von Verbindungen der Formel (I) enthaltenden Zusammensetzungen zur Bekämpfung phytopathogener Schadpilze.

12. Verwendung von Verbindungen der Formel (I) und deren N-Oxiden und landwirtschaftlich unbedenklichen Salzen nach Anspruch 1 und von wenigstens eine Verbindung der Formel (I) enthaltenden Zusammensetzungen zum Schutz von Saatgut, den Wurzeln von Sämlingen und Keimlingen gegen Befall durch phytopathogene Schadpilze und/oder arthropode Schädlinge.

13. Saatgut, enthaltend eine Verbindung der Formel (I) oder ein N-Oxid oder ein landwirtschaftlich unbedenkliches Salz davon nach Anspruch 1 ein einer Menge von 0,1 g bis 10 kg pro 100 kg Saatgut.

## Revendications

1. Composés pyridazin-4-ylméthylsulfonamides représentés par la formule (I) 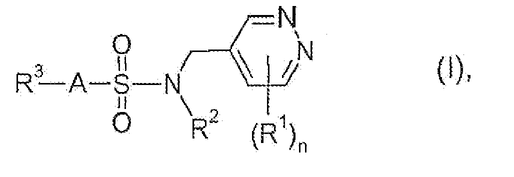 où :
n vaut zéro, un, deux ou trois ;
R¹ est un halogène, un alkyle en C₁-C₄, un halogénoalkyle en C₁-C₄, un alcoxy en C₁-C₄, un halogénoalcoxy en C₁-C₄, un alkylthio en C₁-C₄, un halogénoalkylthio en C₁-C₄, un alkylsulfinyle en C₁-C₄, un halogénoalkylsulfinyle en C₁-C₄, un alkylsulfonyle en C₁-C₄, un halogénoalkylsulfonyle en C₁-C₄, un amino, un alkylamino en C₁-C₄, un di (alkyl en C₁-C₄) amino, un (alkoxy en C₁-C₄) (alkyle en C₁-C₄), un (halogénoalcoxy en C₁-C₄) (alkyle en C₁-C₄), un alcényle en C₂-C₄, un alcynyle en C₂-C₄, un cycloalkyle en C₃-C₃ ou un (alkyl en C₁- C₄) (cycloalkyle en C₃-C₈) ; et/ou un radical R¹ qui est lié à un atome de carbone adjacent à un atome d'azote du noyau pyridazine peut former conjointement avec lesdits atome de carbone et atome d'azote un hétérocycle à cinq chaînons condensé, qui peut contenir un, deux ou trois autres atomes d'azote comme chaînons cycliques ; ou deux radicaux R¹ qui sont liés à des atomes de carbone adjacentes du noyau pyridazine peuvent former conjointement avec lesdits atomes de carbone un noyau benzénique condensé, un carbocycle à 5, 6 ou 7 chaînons saturé ou partiellement insaturé condensé ou un hétérocycle à 5, 6 ou 7 chaînons condensé contenant un, deux ou trois hétéroatomes choisis dans le groupe constitué par 2 atomes d'azote, 1 atome d'oxygène et 1 atome de soufre comme chaînons cyclique, sachant qu'il est possible que le noyau condensé porte un ou deux radicaux choisis dans le groupe constitué par les radicaux halogène, alkyle en C₁- C₄₁ halogénométhyle, alcoxy en C₁-C₄ ou halogénométhoxy ;
R⁴ est l'hydrogène, un alkyle en C₁-C₄, un halogénoalkyle en C₁-C₄, un alcoxy en C₁-C₄, un halogénoalcoxy en C₁-C₄, un alkylamino en C₁-C₄, un di(alkyl en C₁-C₄) amino, un (alcoxy en C₁-C₄) (alkyle en C₁-C₄), (un halogénoalcoxy en C₁-C₄) (alkyle en C₁-C₄), un alcényle en C₂-C₄, un halogénoalcényle en C₂-C₄, un alcynyle en C₂-C₄, un cycloalkyl en C₃- C₈, un (alkyl en C₁-C₄) (cycloalkyle en C₃-C₈) ou un benzyle où la fraction phényle du benzyle est non substituée ou porte 1, 2, 3, 4 ou 5 substituants choisis dans le groupe constitué par les substituants cyano, halogène, alkyle en C₁-C₄, halogénoalkyle en C₁-C₄, alcoxy en C₁-C₄, halogénoalcoxy en C₁-C₄, (alcoxy en C₁-C₄)carbonyle et di (alkyl en C₁-C₄) aminocarbonyle
A est un phénylène, un hétéroarènediyle à 5 ou 6 chaînons, où l'hétéroarènediyle contient un, deux, trois ou quatre hétéroatomes choisis dans le groupe constitué par 1, 2, 3 ou 4 atomes d'azote, 1 atome d'oxygène et 1 atome de soufre, comme chaînons cycliques, et où le phénylène et l'hétéroarènediyle pour leur partie sont non substitués ou portent 1, 2, 3 ou 4 substituants R⁴, chacun choisi dans le groupe constitué par les substituants cyano, halogène, alkyle en C₁-C₄, halogénoalkyle en C₁-C₄, alcoxy en C₁-C₄, halogénoalcoxy en C₁-C₄, alcényle en C₂-C₄, halogénoalcényle en C₂-C₄, alcynyle en C₂-C₄, halogénoalcynyle en C₂-C₄, (alcoxy en C₁- C₄) carbonyle et di (alkyl en C₁-C₄) aminocarbonyle ; ou
A est un alcanediyle en C₁-C₈, un halogénoalcanediyle en C₁-C₈, un alcènediyle en C₂-C₈, un halogénoalcènediyle et C₂-C₈, un alcynediyle en C₂- C₈ ou un halogénoalcynediyle en C₂-C₈, où les six derniers radicaux sont non substitués ou portent un, deux ou trois substituants R^{AA}, chacun choisi dans le groupe constitué par les substituants cyano, alcoxy en C₁-C₄, halogénoalcoxy en C₁-C₄, (alcoxy en C₁₋C₄) carbonyle et di (alkyl en C₁- C₄) aminocarbonyle ;
R³ est l'hydrogène, un alcoxy en C₁-C₄, un halogénoalcoxy en C₁-C₄, un alkylthio en C₁-C₄, un halogénoalkylthio en C₁-C₄, un alkylamino en C₁-C₄, un di (alkyl en C₁-C₄) amino, un (alkyl en C₁- C₄) carbonyle, un (alkoxy en C₁-C₄) iminométhyle, un acryloyl (vinylcarbonyle), un cycloalkyle en C₃-C₈, un (alkyl en C₁-C₄) (cycloalkyle en C₃₋C₈) ou un cycloalcényle en C₅-C₈, un phényle, un benzyle, un phenoxy, un phénylthio, un radical héteroaryle à 5 ou 6 chaînons, où le noyau hétéroaryle a 1, 2, 3 ou 4 hétéroatomes choisis dans le groupe constitué par 1, 2, 3 ou 4 atomes d'azote, 1 atome d'oxygène et 1 atome de soufre, comme chaînons cycliques, sachant qu'il est possible que le noyau hétéroaryle et le noyau phényle du phényle, du benzyle, du phénoxy et du phénylthio soient non substitués ou substitués par un, deux ou trois substituants R^{B}, chacun choisi dans le groupe constitué par les substituants cyano, nitro, amino, halogène, alkyle en C₁-C₄, halogénoalkyle en C₁-C₄, alcoxy en C₁-C₄, halogénoalcoxy en C₁-C₄, (alkoxy en C₁-C₄) carbonyle, di (alkyl en C₁-C₄)aminocarbomyle, alkylthio en C₁- C₄, alkylsulfinyle en C₁-C₄ et alkylsulfonyle en C₁- C₄ ; ou un radical hétéroaryloxy à 5 ou 6 chaînons ou un radical hétéroarylthio à 5 ou 6 chaînons, où le noyau hétéroaryle dans les deux radicaux susmentionnées a 1, 2, 3 ou 4 hétéroatomes choisis dans le groupe constitué par 1, 2, 3 ou 4 atomes d'azote, 1 atome d'oxygène et 1 atome de soufre, comme chaînons cycliques, sachant qu'il est possible que ledit noyau hétéroaromatique porte un, deux ou trois substituants R^{B} ; ou si A est un phénylène ou un hétéroarènediyle à 5 ou 6 chaînons, le radical R³ conjointement avec un radical R^{A} peut former conjointement avec les atomes de carbone auxquels ils sont liés un noyau benzénique condensé, où le noyau benzénique condensé peut être non substitué ou peut porter 1, 2 ou 3 substituants choisis indépendamment les uns des autres dans le groupe constitué par les substituants halogène, cyano, nitro, alkyle en C₁- C₄, halogénoalkyle en C₁-C₄, alcoxy en C₁-C₄, halogénoalcoxy en C₁-C₄, (alcoxy en C₁-C₄) carbonyle et di(alkyl en C₁-C₄)aminocarbonyle, ou si A est un phénylène ou un hétéroarènediyle à 5 ou 6 chaînons, le radical R³ peut également être un allyle en C₁-C₄, un alcényle en C₂-C₄ ou un alcynyle en C₂-C₄ ;
et N-oxydes, sels acceptables du point de vue agricole et sels acceptables du, point de vue vétérinaire des composés représentés par la formule (I), sauf pour le composé *N*-(3,6-dichloropyridazin-4-ylméthyl)-4-méthylbenzènesulfonamide.

2. Composés représentés par la formule (I) selon la revendication 1, dans lesquels A est un phénylène ou un hétéroarènediyle, qui peuvent tous deux être non substitués ou peuvent tous deux porteur un, deux ou trois substituant R^{A}.

3. Composés représentés par la formule (I) selon la revendication 2, dans lesquels A est un 1,3- ou 1,4-phénylène, qui peut porter un, deux ou trois substituant R^{A}.

4. Composés représentés par la formule (I) selon la revendication 2, dans lesquels A est un hétéroarènediyle, choisi dans le groupe constitué par les hétéroarènediyles thiophène-2,5-diàrle, thiophène-2,4-diyle, thiophène-3,5-diyle, thiazole-2,5-diyle, thiasole-2,4-diyle, oxazole-2,5-diyle, oxazole-2,4-diyle, pyrazole-3,5-diyle, pyrazole-1,3-diyle, pyrazole-1,4-diyle, pyridine-2,5-diyle, pyridine-2,6-diyle, pyridine-2,4-diyle, pyridine-3,5-diyle, où l'hétéroarènediyle est non substitué ou porte un, deux ou trois substituants R^{A}.

5. Composés représentés parle formule (I) selon la revendication 1, dans lesquels R³ est un phényle ou un phenoxy, où les deux derniers radicaux sont non substitués ou portent un, deux ou trois substituants R^{B}.

6. Composés représentés par la formule (I) selon la revendication 1, dans lesquels R² est l'hydrogène.

7. Composés représentés par la formule (I) selon l'une quelconque des revendications précédentes, dans lesquels n vaut zéro.

8. Procédé pour la préparation de composés pyridazine-4-ylmétnylsulfonamides représentés par la formule (I) tels que définis dans la revendication 1, qui comprend la réaction d'un composé aminométhylpyridazine représenté par la formule (II) 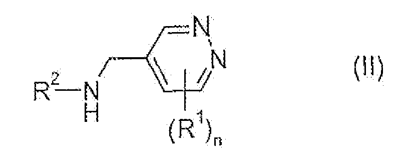 dans laquelle n, R¹ et R² sont tels que définis dans la revendication 1,
dans des conditions basiques avec un dérivé d'acide sulfonique représenté par la formule (III) 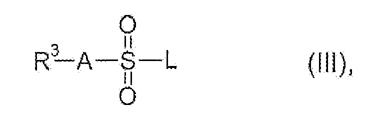 dans laquelle A et R³ sont tels que définis dans la revendication 1 et L est un hydroxy ou un halogène.

9. Composition agricole qui comprend un support solide ou liquide et au moins un composé représenté par la formule (I) ou un N-oxyde ou un sel acceptable du point de vue agricole de celui-ci, selon la revendication 1.

10. Procédé pour le traitement de champignons phytopathogènes nuisibles, lequel procédé comprend le traitement du champignon ou des matériels, des plantes, du sol ou des semences devant être protégés contre une attaque fongique, avec une quantité efficace d'au moins un composé représenté par la formule (I) ou un N-oxyde ou un sel acceptable du point de vue agricole de celui-cri, selon la revendication 1.

11. Utilisation de composés représentés par la formule (I), de leurs N'-oxydes et de leurs sels acceptables du point de vue agricole, selon la revendication 1, et de compositions comprenant des composés de formule (I), pour la lutte contre des champignons phytopathogènes nuisibles.

12. Utilisation de composés représentés par la formule (I) et des N-oxydes et des sels acceptables du point de vue agricole, selon la revendication 1, et de compositions comprenant au moins un composé de formule (I), pour la protection d'une semence, des racines et pousses de semis d'une infestation par des champignons phytopathogènes nuisibles et/ou des anomaux nuisibles arthropodes.

13. Semence comprenant un composé représenté par la formule (I), ou un N-oxvde ou un sel acceptable du point de vue agricole de celui-ci, tel que défini dans la revendication 1, en une quantité de 0,1 g à 10 kg pour 100 kg de semence.
